(19) Europäisches Patentamt
European Patent Office
Office européen des brevets

(11) EP 2 851 423 B1

(12) EUROPEAN PATENT SPECIFICATION

(45) Date of publication and mention of the grant of the patent:
**02.03.2016 Bulletin 2016/09**

(51) Int Cl.:
***C12N 9/80*** *(2006.01)*

(21) Application number: **14173437.6**

(22) Date of filing: **28.12.2006**

(54) **Mutant type II beta-lactam acylases**

Mutierte Type II Beta-Laktam-Acylasen

Acylases de bêta-lactame de type II mutées

(84) Designated Contracting States:
**AT BE BG CH CY CZ DE DK EE ES FI FR GB GR HU IE IS IT LI LT LU LV MC NL PL PT RO SE SI SK TR**

(30) Priority: **28.12.2005 EP 05113024**

(43) Date of publication of application:
**25.03.2015 Bulletin 2015/13**

(62) Document number(s) of the earlier application(s) in accordance with Art. 76 EPC:
**06841245.1 / 1 974 029**

(73) Proprietor: **DSM Sinochem Pharmaceuticals Netherlands B.V.**
**2613 AX Delft (NL)**

(72) Inventors:
- **Van der Laan, Jan Metske**
**6100 AA Echt (NL)**
- **Kerkman, Richard**
**6100 AA Echt (NL)**
- **Bijleveld, Willem**
**6100 AA Echt (NL)**
- **Gielesen, Bianca Elisabeth Maria**
**6100 AA Echt (NL)**

(74) Representative: **De Vroom, Erik**
**DSM Intellectual Property**
**P.O. Box 4**
**6100 AA Echt (NL)**

(56) References cited:

| | |
|---|---|
| **EP-A- 0 558 241** | **EP-A- 1 553 175** |
| **WO-A-91/16435** | **WO-A-95/12680** |
| **WO-A-2005/014821** | **JP-A- H08 205 864** |

- **POLLEGIONI L. ET AL.: "Evolution of an acylase active on cephalosporin C", PROTEIN SCIENCE, vol. 14, no. 12, December 2005 (2005-12), pages 3064-3076, XP009068192,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 851 423 B1

SEQUENCE LISTING

<110> DSM IP Assets B.V

<120> Mutant Acylases

<130> 24988WO

<160> 3

<170> PatentIn version 3.3

<210> 1
<211> 3141
<212> DNA
<213> Pseudomonas sp. SE83

<220>
<221> CDS
<222> (562)..(2886)

<400> 1

```
aagcttgcga tcgcggacgc cgctgccgcc ctgaagaaga ccgcctacaa gggcgagccg      60
gtggtcttcc tgacggtcgc cgggtcgatc tcgcagacgg ccggcgcggt tctggccagc     120
aatatgcgca aggccggctt caccgtggac gagcaggtga tggactgggg cacggtgctc     180
gcacgccggg ccaagaagga cggttggagc gtcttcccgg tctacgccaa cggcatcgac     240
atgatgtcgc cgctgacgca tttctacatc ggcaacaact gcgcgaacta tgccggctgg     300
agctgcgacg ccgtcatcac ccaaaagctc gccgcctatg ccaaggcgcc tgatccggcc     360
acccgcaagc gcatcgcggc cgagatcagg tcgaggccta taaggacacg ccctccgtga     420
tgtggggcca gttcagccgg ccggccggat accgcctgcg cctcaaagac atcgtccagt     480
ccagcttccg atcttctggc agctcacgct cgacgcgtga gctcgtccag atcccgataa     540
gcaacgaggt ccagacagag a atg acg atg gcg gcc aag acc gat cgc gag      591
                        Met Thr Met Ala Ala Lys Thr Asp Arg Glu
                        1               5                   10

gcc ctg cag gcg gcg ctg ccg ccg ctt tcc ggc agc ctc tcc att ccc      639
Ala Leu Gln Ala Ala Leu Pro Pro Leu Ser Gly Ser Leu Ser Ile Pro
                15                  20                  25

gga ttg agc gcg ccg gtc cgt gtc cag cgc gat ggc tgg ggc atc ccg      687
Gly Leu Ser Ala Pro Val Arg Val Gln Arg Asp Gly Trp Gly Ile Pro
            30                  35                  40

cat atc aag gcc tcg ggc gag gcc gat gcc tat cgc gcg ctg ggc ttc      735
His Ile Lys Ala Ser Gly Glu Ala Asp Ala Tyr Arg Ala Leu Gly Phe
        45                  50                  55

gtc cat gcg cag gac cgc ctt ttc cag atg gaa ctg acg cgc cgc aag      783
Val His Ala Gln Asp Arg Leu Phe Gln Met Glu Leu Thr Arg Arg Lys
    60                  65                  70

gcg ctg ggt cgc gcg gcc gaa tgg ctg ggc gcc gag gca gcc gag gcc      831
Ala Leu Gly Arg Ala Ala Glu Trp Leu Gly Ala Glu Ala Ala Glu Ala
75                  80                  85                  90

gat atc ttg gtg cgc cgg ctc ggc atg gaa aaa gtc tgc cgg cgc gat      879
Asp Ile Leu Val Arg Arg Leu Gly Met Glu Lys Val Cys Arg Arg Asp
                95                  100                 105

ttc gag gcc ctg ggt gcc gag gcg aag gac atg ctg cgg gcc tat gtc      927
Phe Glu Ala Leu Gly Ala Glu Ala Lys Asp Met Leu Arg Ala Tyr Val
            110                 115                 120

gcc ggc gtg aac gcg ttc ctg gct tcc ggt gct cct ttg ccc atc gaa      975
Ala Gly Val Asn Ala Phe Leu Ala Ser Gly Ala Pro Leu Pro Ile Glu
        125                 130                 135
```

```
tat ggc ctg ctc ggc gcc gaa ccg gag ccc tgg gaa ccc tgg cac agc     1023
Tyr Gly Leu Leu Gly Ala Glu Pro Glu Pro Trp Glu Pro Trp His Ser
    140                 145                 150

atc gcc gtg atg cgg cgg ctg ggg ctc ctg atg ggc tcc gtc tgg ttc     1071
Ile Ala Val Met Arg Arg Leu Gly Leu Leu Met Gly Ser Val Trp Phe
155                 160                 165                 170

aag ctc tgg cgg atg ctg gcg ctg ccg gtg gtc gga gcc gcg aat gcg     1119
Lys Leu Trp Arg Met Leu Ala Leu Pro Val Val Gly Ala Ala Asn Ala
                175                 180                 185

ctg aag ctg cgc tat gac gat ggc ggc caa gac ctg ctc tgc atc ccg     1167
Leu Lys Leu Arg Tyr Asp Asp Gly Gly Gln Asp Leu Leu Cys Ile Pro
            190                 195                 200

ccg ggt gtc gag gcc gag cgg ctc gaa gcg gat ctc gcg gcg ctg agg     1215
Pro Gly Val Glu Ala Glu Arg Leu Glu Ala Asp Leu Ala Ala Leu Arg
        205                 210                 215

ccc gcg gtt gat gcc ctg ctg aaa gcg atg ggc ggc gac gcc tcc gat     1263
Pro Ala Val Asp Ala Leu Leu Lys Ala Met Gly Gly Asp Ala Ser Asp
    220                 225                 230

gcg gcc ggc ggc ggc agc aac aac tgg gcg gtc gcg ccg ggc cgc acg     1311
Ala Ala Gly Gly Gly Ser Asn Asn Trp Ala Val Ala Pro Gly Arg Thr
235                 240                 245                 250

gcg acg ggc cgg ccc atc ctc gcg ggc gat ccg cat cgc gtc ttc gaa     1359
Ala Thr Gly Arg Pro Ile Leu Ala Gly Asp Pro His Arg Val Phe Glu
                255                 260                 265

atc ccc ggc atg tat gcg cag cat cac ctg gcc tgc gat cgg ttc gac     1407
Ile Pro Gly Met Tyr Ala Gln His His Leu Ala Cys Asp Arg Phe Asp
            270                 275                 280

atg atc ggt ctg acc gtg ccg ggt gtg ccg ggc ttc ccg cat ttc gcg     1455
Met Ile Gly Leu Thr Val Pro Gly Val Pro Gly Phe Pro His Phe Ala
        285                 290                 295

cat aac ggc aag gtc gcc tac tgc gtc acc cat gcc ttc atg gac att     1503
His Asn Gly Lys Val Ala Tyr Cys Val Thr His Ala Phe Met Asp Ile
    300                 305                 310

cac gat ctc tat ctc gag caa ttc gcg gag gac ggg cgc acg gcg cgg     1551
His Asp Leu Tyr Leu Glu Gln Phe Ala Glu Asp Gly Arg Thr Ala Arg
315                 320                 325                 330

ttc ggc aac gag ttc gag ccc gta gcc tgg cgc cga gac cgt atc gcg     1599
Phe Gly Asn Glu Phe Glu Pro Val Ala Trp Arg Arg Asp Arg Ile Ala
                335                 340                 345

gtc cgg ggt ggc gcc gat cgc gaa ttc gat atc gtc gag acg cgc cat     1647
Val Arg Gly Gly Ala Asp Arg Glu Phe Asp Ile Val Glu Thr Arg His
            350                 355                 360

ggc ccc gtc atc gcg ggc gat ccg ctc gag gga gca gcg ctc acg ctg     1695
Gly Pro Val Ile Ala Gly Asp Pro Leu Glu Gly Ala Ala Leu Thr Leu
        365                 370                 375

cgc tcg gtc cag ttc gcc gag acc gac ctt tcc ttc gat tgc ctg acg     1743
Arg Ser Val Gln Phe Ala Glu Thr Asp Leu Ser Phe Asp Cys Leu Thr
    380                 385                 390

cgg atg ccg ggc gca tcg acc gtg gcg cag ctt tac gac gcg acg cgc     1791
Arg Met Pro Gly Ala Ser Thr Val Ala Gln Leu Tyr Asp Ala Thr Arg
395                 400                 405                 410

ggc tgg ggc ctg atc gac cat aat ctc gtc gcc ggg gat gtc gcg ggc     1839
Gly Trp Gly Leu Ile Asp His Asn Leu Val Ala Gly Asp Val Ala Gly
                415                 420                 425

tcg atc ggc cat ctg gtc cgc gcc cgc gtc ccg tcc cgc ccg cgc gag     1887
Ser Ile Gly His Leu Val Arg Ala Arg Val Pro Ser Arg Pro Arg Glu
            430                 435                 440
```

```
aac ggc tgg ctg ccg gtg ccg ggc tgg tcc ggc gag cat gaa tgg cgc      1935
Asn Gly Trp Leu Pro Val Pro Gly Trp Ser Gly Glu His Glu Trp Arg
        445                 450                 455

ggc tgg att ccg cac gag gcg atg ccg cgc gtc atc gat ccg ccg ggc      1983
Gly Trp Ile Pro His Glu Ala Met Pro Arg Val Ile Asp Pro Pro Gly
    460                 465                 470

ggc ctc atc gtc acg gcg aac aac cgc gtc gtg gcc gat gat cat ccc      2031
Gly Leu Ile Val Thr Ala Asn Asn Arg Val Val Ala Asp Asp His Pro
475                 480                 485                 490

gat tat ctc tgt acc gat tgc cat ccg ccc tac cgc gcc gaa cgg atc      2079
Asp Tyr Leu Cys Thr Asp Cys His Pro Pro Tyr Arg Ala Glu Arg Ile
                495                 500                 505

atg gag cgc ctg gtc gcc agt ccg gct ttc gcc gtc gac gat gcg gcc      2127
Met Glu Arg Leu Val Ala Ser Pro Ala Phe Ala Val Asp Asp Ala Ala
            510                 515                 520

gcg atc cac gcc gat acg ctg tcc ccc cat gtc ggc ttg ctg cgc gcg      2175
Ala Ile His Ala Asp Thr Leu Ser Pro His Val Gly Leu Leu Arg Ala
        525                 530                 535

agg ctc gaa gcg ctc gga atc cag ggc agt ctc cct gcc gaa gag ttg      2223
Arg Leu Glu Ala Leu Gly Ile Gln Gly Ser Leu Pro Ala Glu Glu Leu
    540                 545                 550

agg cag acc ctc atc gcc tgg gac ggc cgc atg gat gct ggc tcg cag      2271
Arg Gln Thr Leu Ile Ala Trp Asp Gly Arg Met Asp Ala Gly Ser Gln
555                 560                 565                 570

gcg gct tcc gct tat aat gcg ttc cgc agg gcg ctg acg cgg ctg gta      2319
Ala Ala Ser Ala Tyr Asn Ala Phe Arg Arg Ala Leu Thr Arg Leu Val
                575                 580                 585

acg gcc cgc agc ggg ctg gag caa gcg ata gcg cat ccc ttc gcg gcc      2367
Thr Ala Arg Ser Gly Leu Glu Gln Ala Ile Ala His Pro Phe Ala Ala
            590                 595                 600

gtc ccg ccc ggc gtc tcg ccg cag ggg cag gtc tgg tgg gcc gtg ccg      2415
Val Pro Pro Gly Val Ser Pro Gln Gly Gln Val Trp Trp Ala Val Pro
        605                 610                 615

acc ctg ctg cgc aac gac gat gcc ggg atg ctg aaa ggc tgg agc tgg      2463
Thr Leu Leu Arg Asn Asp Asp Ala Gly Met Leu Lys Gly Trp Ser Trp
    620                 625                 630

gac gag gcc ttg tcg gag gcc ctg tcc gtc gcg acg cag aac ctg acc      2511
Asp Glu Ala Leu Ser Glu Ala Leu Ser Val Ala Thr Gln Asn Leu Thr
635                 640                 645                 650

ggg cgc ggc tgg ggc gag gag cat cgg ccg cgt ttc acg cac ccg ctc      2559
Gly Arg Gly Trp Gly Glu Glu His Arg Pro Arg Phe Thr His Pro Leu
                655                 660                 665

tcc gcg cag ttc ccg gcc tgg gcc gcg ctg ctg aac ccg gtt tcg cgc      2607
Ser Ala Gln Phe Pro Ala Trp Ala Ala Leu Leu Asn Pro Val Ser Arg
            670                 675                 680

ccg atc ggc ggc gat ggc gac acc gtg ctg gcg aac ggg ctc gtc cca      2655
Pro Ile Gly Gly Asp Gly Asp Thr Val Leu Ala Asn Gly Leu Val Pro
        685                 690                 695

tcg gcc gga cct gag gcg acc tat ggc gcc ctg tcg cgc tac gtc ttc      2703
Ser Ala Gly Pro Glu Ala Thr Tyr Gly Ala Leu Ser Arg Tyr Val Phe
    700                 705                 710

gat gtc ggc aat tgg gac aat agc cgc tgg gtc gtc ttc cac ggc gcc      2751
Asp Val Gly Asn Trp Asp Asn Ser Arg Trp Val Val Phe His Gly Ala
715                 720                 725                 730

tcg ggg cat ccg gcc agc ccc cac tat gcc gac cag aat gcg cca tgg      2799
Ser Gly His Pro Ala Ser Pro His Tyr Ala Asp Gln Asn Ala Pro Trp
                735                 740                 745
```

```
agc gac tgc gcg atg gtg ccg atg ctc tat agc tgg gac agg atc gcc      2847
Ser Asp Cys Ala Met Val Pro Met Leu Tyr Ser Trp Asp Arg Ile Ala
            750                 755                 760

gcg gag gcc gtg acc tcg cag gaa ctc gtc ccg gcc tga ggggcaaggc       2896
Ala Glu Ala Val Thr Ser Gln Glu Leu Val Pro Ala
        765                 770

tgcggtcagc ctgccgcagc attcttgcgg caggcgcggg tgcgtaagcc cgctgtttcg    2956

ccgccgtcga cggtcaggac ggcgccgttc acatagctcg acgcgtcgga caggagccag    3016

gccgcgagat cggcgacctc gtccggcgtg ccgagccggc ctgccggaat gcgcagctca    3076

agctgctcca gccgcagcgg gtcggccagc accttgtcca tcatccgcgt cgcgatctgc    3136

ccggg                                                                3141
```

<210> 2
<211> 2847
<212> DNA
<213> Brevundimonas diminuta N-176

<220>
<221> CDS
<222> (483)..(2801)

<400> 2

```
cccggggatc tcgcagacgg ctggcgcggt cctggccagc aatatgcgca aggccggctt     60
cacggtggaa cagcaggtga tggattgggg cacggtgctc gcccgccggg ccaagaagga    120
cggctggagc gttttcccgg tctacgccaa cggcatcgac atgatgtcgc cgctgacgca    180
tttctacatc ggcaacaact gcgtgaacta tgcgggctgg agctgcgacg ccgtcatcac    240
cgaaaagctc gccgcctatg ccaaggcgcc cgatccggct acccgcaaac gcatcgcggc    300
cgaaatccag gtcgaggcct acaaggacac gccctccgtg atgtggggcc agttcagccg    360
gccggcgggc taccgcctgc gcctcaagaa catcgtccag tccagcttcc cgatcttctg    420
gcagctcacg ctcgacgcgt gagcttgccc agattccgac aagcaatgag gtcccgacgc    480
ga atg act atg gcg gcc aac acc gat cgc gcg gtc ttg cag gcg gcg       527
   Met Thr Met Ala Ala Asn Thr Asp Arg Ala Val Leu Gln Ala Ala
   1               5                   10                  15

ctg ccg ccg ctt tcc ggc agc ctc ccc att ccc gga ttg agc gcg tcg       575
Leu Pro Pro Leu Ser Gly Ser Leu Pro Ile Pro Gly Leu Ser Ala Ser
                20                  25                  30

gtc cgc gtc cgg cgc gat gcc tgg ggc atc ccg cat atc aag gcc tcg       623
Val Arg Val Arg Arg Asp Ala Trp Gly Ile Pro His Ile Lys Ala Ser
            35                  40                  45

ggc gag gcc gat gcc tat cgg gcg ctg ggc ttc gtc cat tcg cag gac       671
Gly Glu Ala Asp Ala Tyr Arg Ala Leu Gly Phe Val His Ser Gln Asp
        50                  55                  60

cgt ctt ttc cag atg gag ctg acg cgt cgc aag gcg ctg gga cgc gcg       719
Arg Leu Phe Gln Met Glu Leu Thr Arg Arg Lys Ala Leu Gly Arg Ala
    65                  70                  75

gcc gaa tgg ctg ggc gcc gag gcc gcc gag gcc gat atc ctc gtg cgc       767
Ala Glu Trp Leu Gly Ala Glu Ala Ala Glu Ala Asp Ile Leu Val Arg
80                  85                  90                  95

cgg ctc gga atg gaa aaa gtc tgc cgg cgc gac ttc gag gcc ttg ggc       815
Arg Leu Gly Met Glu Lys Val Cys Arg Arg Asp Phe Glu Ala Leu Gly
                100                 105                 110
```

gtc gag gcg aag gac atg ctg cgg gct tat gtc gcc ggc gtg aac gca 863
Val Glu Ala Lys Asp Met Leu Arg Ala Tyr Val Ala Gly Val Asn Ala
115 120 125

ttc ctg gct tcc ggt gct ccc ctg cct gtc gaa tac gga ttg ctc gga 911
Phe Leu Ala Ser Gly Ala Pro Leu Pro Val Glu Tyr Gly Leu Leu Gly
130 135 140

gca gag ccg gag ccc tgg gag cct tgg cac agc atc gcg gtg atg cgc 959
Ala Glu Pro Glu Pro Trp Glu Pro Trp His Ser Ile Ala Val Met Arg
145 150 155

cgg ctg ggc ctg ctt atg ggt tcg gtg tgg ttc aag ctc tgg cgg atc 1007
Arg Leu Gly Leu Leu Met Gly Ser Val Trp Phe Lys Leu Trp Arg Ile
160 165 170 175

ctg gcg ctg ccg gtg gtc gga gcc gcc aat gcg ctg aag ctg cgc tat 1055
Leu Ala Leu Pro Val Val Gly Ala Ala Asn Ala Leu Lys Leu Arg Tyr
180 185 190

gac gat ggc ggc cgg gat ttg ctc tgc atc ccg ccg ggc gcc gaa gcc 1103
Asp Asp Gly Gly Arg Asp Leu Leu Cys Ile Pro Pro Gly Ala Glu Ala
195 200 205

gat cgg ctc gag gcg gat ctc gcg acc ctg cgg ccc gcg gtc gat gcg 1151
Asp Arg Leu Glu Ala Asp Leu Ala Thr Leu Arg Pro Ala Val Asp Ala
210 215 220

ctg ctg aag gcg atg ggc ggc gat gcc tcc gat gct gcc ggc ggc ggc 1199
Leu Leu Lys Ala Met Gly Gly Asp Ala Ser Asp Ala Ala Gly Gly Gly
225 230 235

agc aac aac tgg gcg gtc gct ccg ggc cgc acg gcg acc ggc agg ccg 1247
Ser Asn Asn Trp Ala Val Ala Pro Gly Arg Thr Ala Thr Gly Arg Pro
240 245 250 255

atc ctc gcg ggc gat ccg cat cgc gtc ttc gaa atc ccg ggc atg tat 1295
Ile Leu Ala Gly Asp Pro His Arg Val Phe Glu Ile Pro Gly Met Tyr
260 265 270

gcg cag cat cat ctg gcc tgc gac cgg ttc gac atg atc ggc ctg acc 1343
Ala Gln His His Leu Ala Cys Asp Arg Phe Asp Met Ile Gly Leu Thr
275 280 285

gtg ccg ggc gtg ccg ggc ttc ccg cac ttc gcg cat aac ggc aag gtc 1391
Val Pro Gly Val Pro Gly Phe Pro His Phe Ala His Asn Gly Lys Val
290 295 300

gcc tat tgc gtc acc cat gcc ttc atg gac atc cac gat ctc tat ctc 1439
Ala Tyr Cys Val Thr His Ala Phe Met Asp Ile His Asp Leu Tyr Leu
305 310 315

gag cag ttc gcg ggg gag ggc cgc act gcg cgg ttc ggc aac gat ttc 1487
Glu Gln Phe Ala Gly Glu Gly Arg Thr Ala Arg Phe Gly Asn Asp Phe
320 325 330 335

gag ccc gtc gcc tgg agc cgg gac cgt atc gcg gtc cgg ggt ggc gcc 1535
Glu Pro Val Ala Trp Ser Arg Asp Arg Ile Ala Val Arg Gly Gly Ala
340 345 350

gat cgc gag ttc gat atc gtc gag acg cgc cat ggc ccg gtt atc gcg 1583
Asp Arg Glu Phe Asp Ile Val Glu Thr Arg His Gly Pro Val Ile Ala
355 360 365

ggc gat ccg cgc gat ggc gca gcg ctc acg ctg cgt tcg gtc cag ttc 1631
Gly Asp Pro Arg Asp Gly Ala Ala Leu Thr Leu Arg Ser Val Gln Phe
370 375 380

gcc gag acc gat ctg tcc ttc gac tgc ctg acg cgg atg ccg ggc gca 1679
Ala Glu Thr Asp Leu Ser Phe Asp Cys Leu Thr Arg Met Pro Gly Ala
385 390 395

tcg acc gtg gcc cag ctc tac gac gcg acg cgc ggc tgg ggc ctg atc 1727
Ser Thr Val Ala Gln Leu Tyr Asp Ala Thr Arg Gly Trp Gly Leu Ile
400 405 410 415

```
gac cat aac ctc gtc gcc ggg gat gtc gcg ggc tcg atc ggc cat ctg      1775
Asp His Asn Leu Val Ala Gly Asp Val Ala Gly Ser Ile Gly His Leu
                420                      425                 430

gtc cgc gcc cgc gtt ccg tcc cgt ccg cgc gaa aac ggc tgg ctg ccg      1823
Val Arg Ala Arg Val Pro Ser Arg Pro Arg Glu Asn Gly Trp Leu Pro
            435                      440                 445

gtg ccg ggc tgg tcc ggc gag cat gaa tgg cgg ggc tgg att ccg cac      1871
Val Pro Gly Trp Ser Gly Glu His Glu Trp Arg Gly Trp Ile Pro His
        450                     455                 460

gag gcg atg ccg cgc gtg atc gat ccg ccg ggc ggc atc atc gtc acg      1919
Glu Ala Met Pro Arg Val Ile Asp Pro Pro Gly Gly Ile Ile Val Thr
    465                     470                 475

gcg aat aat cgc gtc gtg gcc gat gac cat ccc gat tat ctc tgc acc      1967
Ala Asn Asn Arg Val Val Ala Asp Asp His Pro Asp Tyr Leu Cys Thr
480                     485                     490                 495

gat tgc cat ccg ccc tac cgc gcc gag cgc atc atg aag cgc ctg gtc      2015
Asp Cys His Pro Pro Tyr Arg Ala Glu Arg Ile Met Lys Arg Leu Val
                500                     505                 510

gcc aat ccg gct ttc gcc gtc gac gat gcc gcc gcg atc cat gcc gat      2063
Ala Asn Pro Ala Phe Ala Val Asp Asp Ala Ala Ala Ile His Ala Asp
            515                     520                 525

acg ctg tcg ccc cat gtc ggg ttg ctg cgc cgg agg ctc gag gcg ctt      2111
Thr Leu Ser Pro His Val Gly Leu Leu Arg Arg Arg Leu Glu Ala Leu
        530                     535                 540

gga gcc cgc gac gac tcc gcg gcc gaa ggg ctg agg cag atg ctc gtc      2159
Gly Ala Arg Asp Asp Ser Ala Ala Glu Gly Leu Arg Gln Met Leu Val
    545                     550                 555

gcc tgg gac ggc cgc atg gat gcg gct tcg gag gtc gcg tct gcc tac      2207
Ala Trp Asp Gly Arg Met Asp Ala Ala Ser Glu Val Ala Ser Ala Tyr
560                     565                     570                 575

aat gcg ttc cgc agg gcg ctg acg cgg ctg gtg acg gac cgc agc ggg      2255
Asn Ala Phe Arg Arg Ala Leu Thr Arg Leu Val Thr Asp Arg Ser Gly
                580                     585                 590

ctg gag cag gcg ata tcg cat ccc ttc gcg gct gtc gcg ccg ggc gtc      2303
Leu Glu Gln Ala Ile Ser His Pro Phe Ala Ala Val Ala Pro Gly Val
            595                     600                 605

tca ccg caa ggc cag gtc tgg tgg gcc gtg ccg acc ctg ctg cgc gac      2351
Ser Pro Gln Gly Gln Val Trp Trp Ala Val Pro Thr Leu Leu Arg Asp
        610                     615                 620

gac gat gcc gga atg ctg aag ggc tgg agc tgg gac cag gcc ttg tct      2399
Asp Asp Ala Gly Met Leu Lys Gly Trp Ser Trp Asp Gln Ala Leu Ser
    625                     630                 635

gag gcc ctc tcg gtc gcg tcg cag aac ctg acc ggg cga agc tgg ggc      2447
Glu Ala Leu Ser Val Ala Ser Gln Asn Leu Thr Gly Arg Ser Trp Gly
640                     645                     650                 655

gaa gag cat cgg ccg cgc ttc acg cat ccg ctt gcc acg caa ttc ccg      2495
Glu Glu His Arg Pro Arg Phe Thr His Pro Leu Ala Thr Gln Phe Pro
                660                     665                 670

gcc tgg gcg ggg ctg ctg aat ccg gct tcc cgt ccg atc ggt ggc gat      2543
Ala Trp Ala Gly Leu Leu Asn Pro Ala Ser Arg Pro Ile Gly Gly Asp
            675                     680                 685

ggc gat acc gtg ctg gcg aac ggg ctc gtc ccg tca gcc ggg ccg cag      2591
Gly Asp Thr Val Leu Ala Asn Gly Leu Val Pro Ser Ala Gly Pro Gln
        690                     695                 700

gcg acc tat ggt gcc ctg tcg cgc tac gtc ttc gat gtc ggc aat tgg      2639
Ala Thr Tyr Gly Ala Leu Ser Arg Tyr Val Phe Asp Val Gly Asn Trp
    705                     710                 715
```

```
gac aat agc cgc tgg gtc gtc ttc cac ggc gcc tcc ggg cat ccg gcc      2687
Asp Asn Ser Arg Trp Val Val Phe His Gly Ala Ser Gly His Pro Ala
720                 725                 730                 735

agc gcc cat tat gcc gat cag aat gcg ccc tgg agc gac tgt gcg atg      2735
Ser Ala His Tyr Ala Asp Gln Asn Ala Pro Trp Ser Asp Cys Ala Met
                740                 745                 750

gtg ccg atg ctc tat agc tgg gac agg atc gcg gca gag gcc gtg acg      2783
Val Pro Met Leu Tyr Ser Trp Asp Arg Ile Ala Ala Glu Ala Val Thr
            755                 760                 765

tcg cag gaa ctc gtc ccg gcctgagggc cgggcctgtt gtcagcctgc              2831
Ser Gln Glu Leu Val Pro
        770

cgcagctctc ttcggc                                                      2847
```

<210> 3
<211> 2325
<212> DNA
<213> Brevundimonas diminuta V22

<220>
<221> CDS
<222> (1)..(2322)

<400> 3

```
atg act atg gct gcc aac acc gat cgc gcc gtc ttg cag gcg gcg ctg      48
Met Thr Met Ala Ala Asn Thr Asp Arg Ala Val Leu Gln Ala Ala Leu
1               5                   10                  15

ccg ccg ctt tcc ggc agc ctc ccc att ccc gga ttg agc gcg tcg gtc      96
Pro Pro Leu Ser Gly Ser Leu Pro Ile Pro Gly Leu Ser Ala Ser Val
            20                  25                  30

cct atc cag cgc gat gcc tgg ggc atc ccg cat atc aag gcc tcc ggc     144
Pro Ile Gln Arg Asp Ala Trp Gly Ile Pro His Ile Lys Ala Ser Gly
        35                  40                  45

gag gcc gat gcc tat cgc gcg ctg ggc ttc gtc cat gcg cag gac cgc     192
Glu Ala Asp Ala Tyr Arg Ala Leu Gly Phe Val His Ala Gln Asp Arg
    50                  55                  60

ctt ttc cag atg gag ctg acg cgt cgc aag gcg ctg gga cgc gcg gcc     240
Leu Phe Gln Met Glu Leu Thr Arg Arg Lys Ala Leu Gly Arg Ala Ala
65                  70                  75                  80

gaa tgg ctg ggt gcc gag gcc gcc gag gcc gat atc ctc gtg cgc cgg     288
Glu Trp Leu Gly Ala Glu Ala Ala Glu Ala Asp Ile Leu Val Arg Arg
                85                  90                  95

ctc ggt atg gaa aaa gtc tgc cga cgc gat ttc gag gcc ctg ggc gcc     336
Leu Gly Met Glu Lys Val Cys Arg Arg Asp Phe Glu Ala Leu Gly Ala
            100                 105                 110

gag gcg aag gac atg ctc cgg gcc tac gtc gcc ggc gtg aac gca ttc     384
Glu Ala Lys Asp Met Leu Arg Ala Tyr Val Ala Gly Val Asn Ala Phe
        115                 120                 125

ctg gct tcc ggt gtt ccc ctg cct gtc gaa tac gga ttg ctc gga gca     432
Leu Ala Ser Gly Val Pro Leu Pro Val Glu Tyr Gly Leu Leu Gly Ala
    130                 135                 140

gag ccg gag ccc tgg gag cct tgg cac agc atc gcg gtg atg cgc cgg     480
Glu Pro Glu Pro Trp Glu Pro Trp His Ser Ile Ala Val Met Arg Arg
145                 150                 155                 160

ctg ggc ctg ctg atg ggt tcg gtc tgg ttc aag ctc tgg cgg atg ctg     528
Leu Gly Leu Leu Met Gly Ser Val Trp Phe Lys Leu Trp Arg Met Leu
                165                 170                 175

gcg ctg ccg gtg gtc gga gcc gcg aat gcg ctg aag ctg cgc tat gac     576
```

```
Ala Leu Pro Val Val Gly Ala Ala Asn Ala Leu Lys Leu Arg Tyr Asp
            180                 185                 190

gat ggc ggc cgc gat ttg ctc tgc atc ccg ccg cgc gcc gaa gcg gat     624
Asp Gly Gly Arg Asp Leu Leu Cys Ile Pro Pro Arg Ala Glu Ala Asp
        195                 200                 205

cgg ctc gag gcg gat ctc gcg acc ctg cgg ccc gcg gtc gat gcg ctg     672
Arg Leu Glu Ala Asp Leu Ala Thr Leu Arg Pro Ala Val Asp Ala Leu
    210                 215                 220

ctg aag gcg atg ggc ggg gat gcc tca gat gcc gcc ggt ggc ggc agc     720
Leu Lys Ala Met Gly Gly Asp Ala Ser Asp Ala Ala Gly Gly Gly Ser
225                 230                 235                 240

aac aac tgg gcg gtc gcg ccg ggc cgt acg gcg acc ggc cgg ccg atc     768
Asn Asn Trp Ala Val Ala Pro Gly Arg Thr Ala Thr Gly Arg Pro Ile
                245                 250                 255

ctc gcg ggc gat ccg cat cgc gtc ttc cag atc ccc ggc atg tat gcc     816
Leu Ala Gly Asp Pro His Arg Val Phe Gln Ile Pro Gly Met Tyr Ala
            260                 265                 270

cag cat cat ctg gcc tgc gat cgc ttc gac atg atc ggc ctg acc gtg     864
Gln His His Leu Ala Cys Asp Arg Phe Asp Met Ile Gly Leu Thr Val
        275                 280                 285

ccg ggc gtg ccg ggt ttt ccg cat ttc gcg cat aac ggc aag gtc gcc     912
Pro Gly Val Pro Gly Phe Pro His Phe Ala His Asn Gly Lys Val Ala
    290                 295                 300

tac tgc gtc acc cat gcc ttc atg gac att cac gat ctc tac ctt gag     960
Tyr Cys Val Thr His Ala Phe Met Asp Ile His Asp Leu Tyr Leu Glu
305                 310                 315                 320

cag ttc gcg gag gag ggc cgc agg gcg cgg ttc ggc aac gat ttc gag     1008
Gln Phe Ala Glu Glu Gly Arg Arg Ala Arg Phe Gly Asn Asp Phe Glu
                325                 330                 335

ccc gcc gcc tgg agc cgg gac cgt atc gcg gtc cgg ggt ggt gcc gac     1056
Pro Ala Ala Trp Ser Arg Asp Arg Ile Ala Val Arg Gly Gly Ala Asp
            340                 345                 350

cgc gaa ttc gat atc atc gag acg cgc cat ggt ccc gtc ata gca ggc     1104
Arg Glu Phe Asp Ile Ile Glu Thr Arg His Gly Pro Val Ile Ala Gly
        355                 360                 365

gat ccg cgc gat ggc gca gcg ctc acg ctg cgc tcg gtc cag ttc gcc     1152
Asp Pro Arg Asp Gly Ala Ala Leu Thr Leu Arg Ser Val Gln Phe Ala
    370                 375                 380

gag acc gat ctg tcc ttc gat tgc ctg acg cgg atg ccg ggc gca tcg     1200
Glu Thr Asp Leu Ser Phe Asp Cys Leu Thr Arg Met Pro Gly Ala Ser
385                 390                 395                 400

acc gtg gcg cag ctc tac gac gcg acg cgc ggc tgg ggc ctg atc gac     1248
Thr Val Ala Gln Leu Tyr Asp Ala Thr Arg Gly Trp Gly Leu Ile Asp
                405                 410                 415

cat aat ctc gtc gcc ggg gat gtc ggg ggc tcg atc ggc cat ctg gtc     1296
His Asn Leu Val Ala Gly Asp Val Gly Gly Ser Ile Gly His Leu Val
            420                 425                 430

cgc gcc cgt gtc ccg tcc cgc tcg cgc gaa aac ggc tgg ctg ccg gtg     1344
Arg Ala Arg Val Pro Ser Arg Ser Arg Glu Asn Gly Trp Leu Pro Val
        435                 440                 445

ccg ggc tgg tcc ggc gag cat gaa tgg cgg ggt tgg att ccg cac gag     1392
Pro Gly Trp Ser Gly Glu His Glu Trp Arg Gly Trp Ile Pro His Glu
    450                 455                 460

gcg atg ccg cgc gtg atc gat ccg ccg ggc ggc atc atc gtc acg gcg     1440
Ala Met Pro Arg Val Ile Asp Pro Pro Gly Gly Ile Ile Val Thr Ala
465                 470                 475                 480

aat aat cgc gtc gtg gcc gat gac cat ccc gat tat ctc tgc acc gat     1488
```

```
Asn Asn Arg Val Val Ala Asp Asp His Pro Asp Tyr Leu Cys Thr Asp
                485                 490                 495

tgc cat ccg ccc tac cgc gcc gag ccc atc atg aag cgc ctg gtc gcc       1536
Cys His Pro Pro Tyr Arg Ala Glu Pro Ile Met Lys Arg Leu Val Ala
            500                 505                 510

aat ccg gct ttc gcc gtc gac gat gcc gcc gcg atc cat gcc gat acg       1584
Asn Pro Ala Phe Ala Val Asp Asp Ala Ala Ala Ile His Ala Asp Thr
        515                 520                 525

ctg tcg ccc cat gtc ggg ttg ctg cgc cgg agg ctc gag gcg ctt gga       1632
Leu Ser Pro His Val Gly Leu Leu Arg Arg Arg Leu Glu Ala Leu Gly
    530                 535                 540

gcc cgc gac gac tcc gcg gcc gaa ggg ctg agg cag atg ctc gtc gcc       1680
Ala Arg Asp Asp Ser Ala Ala Glu Gly Leu Arg Gln Met Leu Val Ala
545                 550                 555                 560

tgg gac ggc cgc atg gat gcg gct tcg gag gtc gcg tct gcc tac aat       1728
Trp Asp Gly Arg Met Asp Ala Ala Ser Glu Val Ala Ser Ala Tyr Asn
                565                 570                 575

gcg ttc cgc agg gcg ctg acg cgg ctg gtg acg gac cgc agc ggg ctg       1776
Ala Phe Arg Arg Ala Leu Thr Arg Leu Val Thr Asp Arg Ser Gly Leu
            580                 585                 590

gag cag gcg ata tcg cat ccc ttc gcg gct gtc gcg ccg ggc gtc tca       1824
Glu Gln Ala Ile Ser His Pro Phe Ala Ala Val Ala Pro Gly Val Ser
        595                 600                 605

ccg caa ggc cag gtc tgg tgg gcc gtg ccg acc ctg ctg cgc gac gac       1872
Pro Gln Gly Gln Val Trp Trp Ala Val Pro Thr Leu Leu Arg Asp Asp
    610                 615                 620

gat gcc gga atg ctg aag ggc tgg agc tgg gac cag gcc ttg tct gag       1920
Asp Ala Gly Met Leu Lys Gly Trp Ser Trp Asp Gln Ala Leu Ser Glu
625                 630                 635                 640

gcc ctc tcg gtc gcg tcg cag aac ctg agc cgg cga agc tgg ggc gaa       1968
Ala Leu Ser Val Ala Ser Gln Asn Leu Ser Arg Arg Ser Trp Gly Glu
                645                 650                 655

gag cat cgg ccg cgc ttc acg cat ccg ctt gcc acg caa ttc ccg gcc       2016
Glu His Arg Pro Arg Phe Thr His Pro Leu Ala Thr Gln Phe Pro Ala
            660                 665                 670

tgg gcg ggg ctg ctg aat ccg gct tcc cgt ccg atc ggc ggc gat ggc       2064
Trp Ala Gly Leu Leu Asn Pro Ala Ser Arg Pro Ile Gly Gly Asp Gly
        675                 680                 685

gac acc gtg ctg gcg aac ggg ctc gtc ccg tca gcc ggg ccg cag gcg       2112
Asp Thr Val Leu Ala Asn Gly Leu Val Pro Ser Ala Gly Pro Gln Ala
    690                 695                 700

acc tat ggc gcc ctg tcg cgc tac gtc ttt gat gtc ggc aat tgg gac       2160
Thr Tyr Gly Ala Leu Ser Arg Tyr Val Phe Asp Val Gly Asn Trp Asp
705                 710                 715                 720

aat agc cgc tgg gtc gtc ttc cac ggc gcc tcc ggg cat ccg gcc agc       2208
Asn Ser Arg Trp Val Val Phe His Gly Ala Ser Gly His Pro Ala Ser
                725                 730                 735

gcc cat tat gcc gat cag aat gcg ccc tgg agc gac tgt gcg atg gtg       2256
Ala His Tyr Ala Asp Gln Asn Ala Pro Trp Ser Asp Cys Ala Met Val
            740                 745                 750

ccg atg ctc tat agc tgg gac agg atc gcg gca gag gcc gtg acg tcg       2304
Pro Met Leu Tyr Ser Trp Asp Arg Ile Ala Ala Glu Ala Val Thr Ser
        755                 760                 765

cag gaa ctc gtc ccg gcc tga                                           2325
Gln Glu Leu Val Pro Ala
    770
```

## Description

The present invention relates to mutant type II beta-lactam acylases, to polynucleotides encoding said enzymes and to microorganisms transformed with said polynucleotides as well as to methods of producing the mutant type II beta lactam. The invention furthermore relates to a process for the production of a deacylated beta-lactam compound of interest using the mutant type II beta lactam acylases of the invention.

Beta-lactam antibiotics constitute the most important group of antibiotic compounds with a long history of clinical use. Among this group, the prominent ones are the penicillins and cephalosporins. Penicillins are naturally produced by various filamentous fungi such as *Penicillium* (e.g. *P. chrysogenum*). Cephalosporins are naturally produced by various microorganisms such as *Acremonium* (e.g. *A. chrysogenum*) and *Streptomyces* (e.g. *Streptomyces clavuligerus*).

As a result of classical strain improvement techniques, the production levels of the antibiotics in *P. chrysogenum* and *A. chrysogenum* have increased remarkably over the past decades. With the increasing knowledge of the biosynthetic pathways leading to penicillins and cephalosporins and the advent of recombinant DNA technology, new tools for the improvement of production strains have become available.

Most enzymes involved in beta-lactam biosynthesis have been identified and their corresponding genes have been cloned as can be found in Ingolia and Queener, Med Res Rev (1989) 9:245-264 (biosynthesis route and enzymes) and Aharonowitz, Cohen, and Martin, Ann Rev Microbiol (1992) 46:461-495 (gene cloning).

The first two steps in the biosynthesis of penicillin in *P. chrysogenum* are the condensation of the three amino acids L-5-amino-5-carboxypentanoic acid (L-alphalpha-aminoadipic acid) (A), L-cystein (C) and L-valine (V) into the tripeptide LLD-ACV, followed by cyclization of this tripeptide to form isopenicillin N. This compound contains the typical beta-lactam structure. The third step involves the replacement of the hydrophilic side chain of L-5-amino-5-carboxypentanoic acid by a hydrophobic side chain by the action of the enzyme acyltransferase (AT).

In EP-A-0448180 it has been described that the enzymatic exchange reaction mediated by AT takes place inside a cellular organelle, the microbody. The observation that substantial quantities of deacetoxycephalosporin C (DAOC) can be formed by non-precursed *P. chrysogenum* transformants expressing deacetoxycephalosporin C synthase (EC 1.14.20.1 - DAOCS, further indicated herein as expandase) implies the presence of significant amounts of penicillin N, the natural substrate for expandase, in *P. chrysogenum* (Alvi et al., J Antibiot (1995) 48:338-340). However, the D-alpha-amino-adipyl side chains of DAOC cannot be easily removed.

Cephalosporins are much more expensive than penicillins. One reason is that some cephalosporins (e.g. cephalexin) are made from penicillins by a number of chemical conversions. Another reason is that, so far, only cephalosporins with a D-alpha-amino-adipyl side chain could be fermented. Cephalosporin C, by far the most important starting material in this respect, is very soluble in water at any pH, thus implying lengthy and costly isolation processes using cumbersome and expensive column technology. Cephalosporin C obtained in this way has to be converted into therapeutically used cephalosporins by a number of chemical and enzymatic conversions.

The methods currently favoured in industry to prepare the intermediate 7-amino-deacetoxycephaloporanic acid (7-ADCA) involve complex chemical steps leading to the expansion and derivatization of penicillin G. One of the necessary chemical steps to produce 7-ADCA involves the expansion of the 5-membered penicillin ring structure to a 6-membered cephalosporin ring structure (see for instance US 4,003,894). This complex chemical processing is both expensive and noxious to the environment. Consequently, there is a great desire to replace such chemical processes with enzymatic reactions such as enzymatic catalysis, preferably during fermentation. A key to the replacement of the chemical expansion process by a biological process is the central enzyme in the cephalosporin biosynthetic pathway, expandase. The expandase enzyme from the bacterium *Streptomyces clavuligerus* (*S. clavuligerus*) was found to carry out, in some cases, penicillin ring expansions. When introduced into *P. chrysogenum*, it can convert the penicillin ring structure into the cephalosporin ring structure, as described in Cantwell et al., Proc R Soc Lond B (1992) 248:283-289. Since the expandase enzyme catalyses the expansion of the 5-membered thiazolidine ring of penicillin N to the 6-membered dihydrothiazine ring of DAOC, this enzyme would be of course a logical candidate to replace the ring expansion steps of the chemical process. Unfortunately, the enzyme works on the penicillin N intermediate of the cephalosporin biosynthetic pathway, but not or very inefficiently on the readily available inexpensive penicillins as produced by *P. chrysogenum,* like penicillin V or penicillin G. Penicillin N is commercially not available and even when expanded, its D-alpha-aminoadipyl side chain cannot be easily removed by penicillin acylases.

It has been reported that the expandase enzyme is capable of expanding penicillins with particular side chains to the corresponding 7-ADCA derivative. This feature of the expandase has been exploited in the technology as disclosed in WO93/05158, WO95/04148 and WO95/04149. In these disclosures the conventional chemical in vitro conversion of penicillin G to 7-ADCA has been replaced by the *in vivo* conversion of certain 6-aminopenicillanic acid (6-APA) derivatives in recombinant *Penicillium chrysogenum* strains transformed with an expandase gene. More particularly, WO93/05158 teaches the *in vivo* use of the expandase enzyme in *P. chrysogenum,* in combination with an adipyl side chain (further referred to as adipyl) as a feedstock, which is a substrate for the acyltransferase enzyme in *P. chrysogenum.* This leads to the formation of adipyl-6-APA, which is converted by an expandase enzyme introduced into the *P. chrysogenum* strain to yield adipyl-7-ADCA which is excreted by the fungal cells into the surrounding medium.

In a subsequent step, the side chains of the corresponding 7-ADCA derivatives can be cleaved off either chemically or enzymatically by an acylase enzyme thus yielding 7-ADCA and the corresponding side chain. Various types of microorganisms have been proposed in the literature as acylase producing strains useful for the deacylation of beta-lactam derivatives obtained by fermentation. Examples of such acylase-producing microorganisms are certain strains of the species *Escherichia coli*, *Kluyvera citrophila, Proteus rettgeri, Pseudomonas sp., Alcaligenes faecalis, Bacillus megaterium, Bacillus sphaericus* and *Arthrobacter viscosus.*

According to the literature several types of acylases may be envisaged, based on their molecular structure and substrate specificity (Vandamme E. J. Penicillin acylases and beta-lactamases" In: "Microbial Enzymes and Bioconversions" E. H. Rose (Ed.), Economic Microbiology 5 (1980) 467-552, Acad. Press, New York).

Type-I acylases are specific for Penicillin V. These enzymes are composed of four identical subunits, each having a molecular weight of 35 kDa. A complete nucleotide sequence of the cloned gene from *Bacillus sphaericus* has been reported (Ollson A. Appl. Environm. Microb. (1976), 203).

Type-II acylases all share a common molecular structure: these enzymes are heterodimers composed of a small alpha-subunit (20-25 kDa) and a large beta-subunit (60-65 kDa). With respect to the substrate specificity, Type-II acylases may be further divided into two groups

Type-IIA acylases are very specific for Penicillin G and therefore generally known as penicillin acylases. In general, they are not so much specific for the moiety adjacent to the nitrogen atom of the amide group (this might be a cephem group, a penem group, an amino acid, etc.), but the substrate specificity resides in the acyl moiety of the substrate. This acyl moiety must be very hydrophobic and is preferably benzyl or (short) alkyl. Examples of substrates that are not hydrolyzed by Type-IIA acylases are those with dicarboxylic acids as acyl moiety: succinyl, glutaryl, adipyl and aminoadipyl, the side-chain of CefC. Examples of Type-IIA acylases are the enzymes from *Escherichia coli, Kluyvera citrophila, Proteus rettgeri* and *Alcaligenes faecalis.*

Type-IIB acylases have been reported to be capable of hydrolyzing cephalosporins (including the desacetoxy-derivative) with succinyl, glutaryl, adipyl and $\alpha$-ketoadipyl as an acyl moiety and even CefC to a very limited degree. The group of Type-IIB acylases again can be divided into two groups on the basis of amino acid sequence homology. These subgroups will be defined here as the SY77-group and SE83-group and are named after the acylase from *Pseudomonas* SY77 and *Pseudomonas* SE83-acylI respectively.

Matsuda et al (J. Bacteriol (1985), 163, 1222 have cloned and sequenced the gene encoding the SY77-acylase and demonstrated that the enzyme was active towards glutaryl-7ACA but much less toward succinyl-7ACA and adipyl-7ACA. The 3-dimensional structure for the SY77-precursor is known (J. Biol.Chem. (2002), 277, 2823).

Later, Matsuda et al. (J. Bacteriol (1987), 169, 5815 and J. Bacteriol. (1987), 169, 5821) cloned and sequenced the gene encoding the SE83-acylI acylase and demonstrated that this enzyme was active towards (in decreasing order) glutaryl-7ACA, adipyl-7ACA, succinyl-7ACA and CEFC (Cephalosporin C). All studies related to SE83 focused on the capacity of the enzyme to hydrolyse derivatives of 7-ACA, in particular to hydrolyse CEFC.

In WO91/16435 it has been shown that the amino acid homology between SY77 and SE83-acylI is very low: approximately 25% for the alpha-subunits and 28% for the beta-subunits of the acylases.

WO9512680 discloses another SE83-group acylase from *Brevundimonas diminuta*, named N176, which is approximately 94% homologous to SE83-acylI and which was tested for its CEFC-acylase activity. A third member of the SE83-group is V22 from *Brevundimonas diminuta* V22. The amino acid sequences of these three acylases are disclosed by Aramori et al in Journal of Fermentation and Bioengineering 72, 232-243 (1991). Table 0 shows the full length sequence identity matrix of the amino acid sequences of the various Type-IIB acylases of the SE83-group.

Table 0.

| Type-IIB acylase | SE83acyii | N176 | V22 |
|---|---|---|---|
| SE83acyii | 100 | 94 | 93 |
| N176 | 94 | 100 | 98 |
| V22 | 93 | 98 | 100 |

Several attempts have been made to increase the CEFC-acylase activity of a few existing acylases: WO2005014821 discloses mutants of the SE83-acylase and EP-A-1553175 discloses mutants of the N176-acylase, all in order to improve the CEFC-acylase activity. None of the cited references focused on the improvement of the deacylation reaction with other acylated beta-lactam compounds of interest such as adipyl-7-ADCA. Therefore, there is still an urgent need for an acylase with an improved deacylating activity towards adipyl-7ADCA and which can be used advantageously in a process for the production of 7-ADCA from adipyl-7-ADCA produced for instance by fermentation of a transformed *Penicillium* strain.

Figure 1: Multiple alignment of the amino acid sequences of the type II beta-lactam acylases SE83-acyii from *Pseudomonas* SE83 (SEQ ID No.1), N176 from *Brevundimonas diminuta* N-176 (SEQ ID No. 2) and V22 from *Brevundimonas diminuta* V22 (SEQ ID No. 3).

Figure 2: Conversion of adipyl-7ADCA by immobilized acylase at pH=8.8 and 30°C (Figure 2a) and at pH=9.5 and 40°C (Figure 2b). *Peudomonas* SE83 ACYii wild type immobilized acylase (solid line) and mutant L161T immobilized acylase (dashed line). The rate (ml KOH/min on the Y-axis) is plotted as a function of the percentage conversion (% on the X-axis).

In a first aspect, the present invention provides a mutant type II beta-lactam acylase that is a variant of a model polypeptide with type II beta-lactam acylase activity whereby the mutant beta-lactam acylase has an at least 1.5-fold improved in vitro beta-lactam acylase activity towards adipyl-7-ADCA in comparison with the model polypeptide with beta-lactam acylase activity. The determination of the in vitro beta-lactam acylase activity towards adipyl-7-ADCA is described in detail in the Materials and Methods section. More preferably the in vitro beta-lactam acylase activity towards adipyl-7-ADCA of the mutant type II beta-lactam acylase is improved at least 2-fold, more preferably at least 2.5-fold, more preferably at least 3-fold, more preferably at least 4-fold, more preferably at least 5-fold, more preferably at least 6-fold, more preferably at least 7-fold, more preferably at least 8-fold, more preferably at least 9-fold, more preferably at least 10-fold, more preferably at least 11-fold.

With "altered or mutant type II beta-lactam acylase" in the context of the present invention is meant any enzyme having acylase activity, which has not been obtained from a natural source and for which the amino acid sequence differs from the complete amino acid sequences of the natural type II beta-lactam acylase enzymes.

Highly preferred embodiments of the present invention are mutants of the model type II beta-lactam acylases selected from the group consisting of the acylase having the amino acid sequence according to SEQ ID NO: 1 and the acylase having the amino acid sequence according to SEQ ID NO: 2 and the acylase having the amino acid sequence according to SEQ ID NO:3 and polypeptides with type II beta-lactam acylase activity having an amino acid sequence with a percentage identity with SEQ ID NO: 1 of at least 70%, preferably at least 75%, more preferably at least 80%, more preferably at least 85%, more preferably at least 90%, most preferably at least 95%, or with SEQ ID NO: 2 of at least 70%, preferably at least 75%, more preferably at least 80%, more preferably at least 85%, more preferably at least 90%, most preferably at least 95%, or with SEQ ID NO: 3 of at least 70%, preferably at least 75%, more preferably at least 80%, more preferably at least 85%, more preferably at least 90%, most preferably at least 95% and which carry one of the following modifications: L161G, L161S or L161T.

The invention also provides a mutant type II beta-lactam acylase that is a variant of a model polypeptide with type II beta-lactam acylase activity whereby the mutant type II beta-lactam acylase has, in addition to position 161, been modified at at least an amino acid position selected from the group consisting of positions 270, 296, 442 and 589 or from the group consisting of positions 10, 29, 274, 280, 314, 514, 645, 694, 706 and 726 or from the group consisting of 10, 29, 270, 274, 280, 296, 314, 442, 514, 589, 645, 694, 706 and 726 or from the group consisting of 10, 29, 270, 274, 280, 442, 514, 589, 645, 694 and 726 using the amino acid position numbering of the amino acid sequence of the SE83-acyll acylase of *Pseudomonas* (SEQ ID NO: 1).

More preferably, the present invention provides a mutant type II beta-lactam acylase that is a variant of a model polypeptide with type II beta-lactam acylase activity whereby the mutant beta-lactam acylase has an at least 1.5-fold improved in vitro beta-lactam acylase activity towards adipyl-7-ADCA in comparison with the model polypeptide with beta-lactam acylase activity, more preferably at least 2-fold, more preferably at least 2.5-fold, more preferably at least 3-fold, more preferably at least 4-fold, more preferably at least 5-fold, more preferably at least 6-fold, more preferably at least 7-fold, more preferably at least 8-fold, more preferably at least 9-fold, more preferably at least 10-fold, more preferably at least 11-fold and whereby the mutant type II beta-lactam acylase has, in addition to position 161, been modified at at least an amino acid position selected from group consisting of positions 270, 296, 442 and 589, or from the group consisting of positions 10, 29, 274, 280, 314, 514, 645, 694, 706 and 726 or from the group consisting of 10, 29, 270, 274, 280, 296, 314, 442, 514, 589, 645, 694, 706 and 726 or from the group consisting of 10, 29, 270, 274, 280, 442, 514, 589, 645, 694 and 726 using the amino acid position numbering of the amino acid sequence of the SE83-acyll acylase of Pseudomonas (SEQ ID NO: 1).

The present invention also provides a mutant type II beta-lactam acylase that is a variant of a model polypeptide with type II beta-lactam acylase activity whereby the mutant type II beta-lactam acylase has been modified at least at a combination of positions 161+270 or at least at a combination of positions 161+296 or at least at a combination of positions 161+442 or at least at a combination of positions 161+589 or at least at a combination of positions 161+270+296 or at least at a combination of positions 161+270+442 or at least at a combination of positions 161+270+589 or at least at a combination of positions 161+296+589 or at least at a combination of positions 161+296+442 or at least at a combination of positions 161+296+589 or at least at the combination of positions 161, 270, 296, 442 and 589 and whereby the mutant type II beta-lactam acylases may have modifications at other amino acid positions in addition to those positions and all possible combinations thereof as described before using the amino acid position numbering of the amino acid sequence of the SE83-acyll acylase of Pseudomonas (SEO ID NO: 1).

The model polypeptide with type II beta-lactam acylase activity as used in the present invention is selected from the group consisting of a polypeptide with type II beta-lactam acylase activity, preferably having an amino acid sequence according to SEQ ID NO: 1 (i.e. the SE83-acyll acylase of *Pseudomonas* species SE83) or having an amino acid sequence according to SEQ ID NO: 2 (i.e. the N176-acylase of *Pseudomonas* species N176) or having an amino acid sequence according to SEQ ID NO:3 (i.e. the V22 acylase of *Brevundimonas diminuta* V22) and polypeptides with type II beta-lactam acylase activity having an amino acid sequence with a percentage identity with SEQ ID NO: 1 of at least 70%, preferably at least 75%, more preferably at least 80%, more preferably at least 85%, more preferably at least 90%, most preferably at least 95%, or with SEQ ID NO: 2 of at least 70%, preferably at least 75%, more preferably at least 80%, more preferably at least 85%, more preferably at least 90%, most preferably at least 95%, %, or with SEQ ID NO: 3 of at least 70%, preferably at least 75%, more preferably at least 80%, more preferably at least 85%, more preferably at least 90%, most preferably at least 95%. More preferred as model polypeptide with type II beta-lactam acylase activity as used in the present invention is a polypeptide with type II beta-lactam acylase activity, having the amino acid sequence according to SEQ ID NO: 1 or having the amino acid sequence according to SEQ ID NO: 2 or having the amino acid sequence according to SEQ ID NO: 3. Most preferred as model polypeptide with type II beta-lactam acylase activity is the SE83-acyll acylase of Pseudomonas (SEQ ID NO: 1).

The present invention preferably provides mutants of the model type II beta-lactam acylases selected from the group consisting of the acylase having the amino acid sequence according to SEQ ID NO: 1 and the acylase having the amino acid sequence according to SEQ ID NO: 2 and the acylase having the amino acid sequence according to SEQ ID NO:3 and polypeptides with type II beta-lactam acylase activity having an amino acid sequence with a percentage identity with SEQ ID NO: 1 of at least 70%, preferably at least 75%, more preferably at least 80%, more preferably at least 85%, more preferably at least 90%, most preferably at least 95%, or with SEQ ID NO: 2 of at least 70%, preferably at least 75%, more preferably at least 80%, more preferably at least 85%, more preferably at least 90%, most preferably at least 95%, %, or with SEQ ID NO: 3 of at least 70%, preferably at least 75%, more preferably at least 80%, more preferably at least 85%, more preferably at least 90%, most preferably at least 95% and whereby the mutants, in addition to a modification at position 161, have modifications at least at position 10 or at least at position 29, at least at position 161 or at least at position 270 or at least at position 274 or at least at position 280 or at least at position 296 or at least at position 314 or at least at position 442 or at least at position 514 or at least at position 589 or at least at position 645 or at least at position 694 or at least at position 706 or at least at position 726. In one embodiment the invention provides mutant type II beta-lactam acylases have a single modification at either position 161 or at position 296 using the amino acid position numbering of the amino acid sequence of the SE83-acyll acylase of Pseudomonas (SEQ ID NO: 1).

Table 1

| Amino Acid | 3-letter code | 1-letter code |
|---|---|---|
| Alanine | Ala | A |
| Arginine | Arg | R |
| Asparagine | Asn | N |
| Aspartic acid | Asp | D |
| Cystein | Cys | C |
| Glutamic acid | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |

(continued)

| Amino Acid | 3-letter code | 1-letter code |
|---|---|---|
| Proline | Pro | P |
| Serine | Ser | S |
| Threonine | Thr | T |
| Tryptophane | Trp | W |
| Tyrosine | Tyr | Y |
| Valine | Val | V |

The modification at an amino acid position may comprise a substitution by another amino acid, selected from the group of 20 L-amino acids that occur in Nature - see Table 1. Alternatively, the modification at an amino acid position may comprise a deletion of the amino acid at said position. Furthermore, the modification at an amino acid position may comprise a substitution of one or more amino acids at the C-terminal or N-terminal side of said amino acid.

The mutant type II beta-lactam acylase of the invention, preferably the mutants of the model type II beta-lactam acylases selected from the group consisting of the acylase having the amino acid sequence according to SEQ ID NO: 1 and the acylase having the amino acid sequence according to SEQ ID NO: 2 and the acylase having the amino acid sequence according to SEQ ID NO:3 carry a substitution of the leucine at position 161 by a smaller and more polar amino acid such threonine, serine, glycine and cystein or a positively charged around pH=9 such as arginine and lysine, preferably by serine or threonine or glycine, most preferably by threonine and may carry one or more of the following modifications:

- Substitution of the glutamate (SEQ ID NO:1) or alanine (SEQ ID NO:2 and SEQ ID NO:3) at position 10 by a positively charged amino acid residue such as lysine or arginine or a small amino acid residue with conformational preference for $\alpha$-helix formation such as alanine, preferably by lysine
- Substitution of the serine at position 29 by an amino acid with an aromatic (like) side chain such as phenylalanine, tyrosine, tryptophane and histidine or a larger uncharged polar or positively charged side chain such as asparagine, glutamine, arginine and lysine, preferably by asparagine or phenylalanine,
- Substitution of the histidine at position 274 by an amino acid residue that contains at least a carbon, oxygen or sulphur atom at the gamma position of the side chain and which is smaller in size compared to histidine such as leucine, isoleucine, cystein, threonine, serine, asparagine, valine and proline, preferably by leucine, isoleucine, cystein or threonine,
- Substitution of the arginine at position 280 by an amino acid residue that substitutes the positive charge by a negative charge such as aspartic acid and glutamic acid or by an unbranched and uncharged polar side chain such as glutamine, asparagine and serine, preferably glutamine and asparagine, most preferably glutamine.
- Substitution of the histidine at position 296 by a charged or polar amino acid or an amino acid residue which is able to replace the in the model acylase existing hydrogen bonding to the N-delta or N-epsilon atoms of the histidine residue such as by asparagine and glutamine, preferably by a glutamine
- Substitution of the isoleucine at position 314 by a smaller amino acid residue with $\beta$-branching such as valine or by a medium size polar side chain such as glutamine, asparagine serine and threonine, preferably by valine or glutamine.
- Substitution of the glutamic acid at position 442 by an amino acis residue with no or a small hydrophobic side chain such as glycine, alanine, leucine, valine and isoleucine, preferably glycine
- Substitution of the proline at position 514 by an amino acid residue with a more polar and/or more flexible side chain which is able to contribute to additional hydrogen bonding such as glutamine, asparagine, threonine, serine, cystein, aspartic acid and glutamic acid, preferably glutamine
- Substitution of the arginine at position 589 by an amino acid residue that can maintain a positive charge in a certain environment such as histidine and lysine or by aromatic side chains able to form hydrogen bonds such as tyrosine and tryptophane or by amino acid residues which are able to replace the in the model acylase existing hydrogen bonding to the N-delta or N-epsilon atoms of the histidine residue such as by asparagine and glutamine, preferably by histidine.
- Substitution of the alanine at position 645 by a small amino acid residue with an increased preference for $\beta$-strand formation such as threonine, valine, serine, cystein and leucine, preferably by threonine
- Substitution of the asparagine at position 694 by an amino acid residue with a side chain smaller than asparagine such as alanine, threonine, serine, cystein, valine and glycine, preferably by a threonine.
- Substitution of the tyrosine at position 706 by an amino acid residue with no or a side chain smaller than leucine, such as glycine, alanine, valine, serine, cystein, threonine and proline, preferably a glycine

- Substitution of the valine at 726 by an amino acid residue with a larger hydrophobic side chain such as isoleucine, leucine and methionine, preferably an isoleucine.

More highly preferred embodiments of the present invention are mutants of the model type II beta-lactam acylases selected from the group consisting of the acylase having the amino acid sequence according to SEQ ID NO: 1 and the acylase having the amino acid sequence according to SEQ ID NO: 2 and the acylase having the amino acid sequence according to SEQ ID NO:3 and polypeptides with type II beta-lactam acylase activity having an amino acid sequence with a percentage identity with SEQ ID NO: 1 of at least 70%, preferably at least 75%, more preferably at least 80%, more preferably at least 85%, more preferably at least 90%, most preferably at least 95%, or with SEQ ID NO: 2 of at least 70%, preferably at least 75%, more preferably at least 80%, more preferably at least 85%, more preferably at least 90%, most preferably at least 95%, %, or with SEQ ID NO: 3 of at least 70%, preferably at least 75%, more preferably at least 80%, more preferably at least 85%, more preferably at least 90%, most preferably at least 95% and which carry a modification at the following combinations of 2 positions: [161+10], [161+29] or [161+694] or [161+726] or [161+274] or [161+706] or [161 +442] or [161 +589] or [161 +314] or have modifications at the following combinations of 3 positions: [161+29+274], [161+29+706], [161+29+514], [161+274+589] or [161+274+706] or at the following combinations of 4 positions: [161+29+274+726], [161+274+280+314], or the following combinations at 5 positions: [161+29+274+314+694], [161+274+280+514+726] or the following combinations of 6 positions: [161+29+280+314+645+726].

The present invention preferably provides mutants of the SE83-acylI acylase of *Pseudomonas* (SEQ ID NO: 1) that have modifications at least at a combination of positions L161+M270 or at least at a combination of positions L161+H296 or at least at a combination of positions L161+E442 or at least at a combination of positions L161+R589 or at least at a combination of positions L161+M270+H296 or at least at a combination of positions L161+M270+E442 or at least at a combination of positions L161+M270+R589 or at least at a combination of positions L161+H296+R589 or at least at a combination of positions L161+H296+E442 or at least at a combination of positions L161+H296+R589 and whereby the mutant type II beta-lactam acylases may have modifications at other amino acid positions in addition to those positions and all possible combinations thereof as described before. Highly preferred embodiments of the present inventions are the *Pseudomonas* SE83-AcylI mutants summarized in Tables 2-5 of the Examples.

In a second aspect, the invention provides a polynucleotide encoding the mutant type II beta-lactam acylase of the present invention. The invention provides also polynucleotides encoding the alpha-subunit of the mutant type II beta-lactam acylase as well as polynucleotides encoding the beta-subunit of the mutant type II beta-lactam acylase. WO2005/014821 discloses on page 8 and 9 that genes encoding the SE83-group acylases are encoding a polypeptide composed of an $\alpha$-subunit, a spacer peptide, and a $\beta$-subunit, in that order. The acylase derived from *Pseudomonas* sp. SE83 is generated in the form of an inactive single chain polypeptide having about 84 kDa in size after undergoing transcription and translation in a host cell. After then, two self-digestions occur between the amino acids at position 230 and 231, and at position 239 and 240 in the amino acid sequence of SEQ ID NO: 1, which results in removing the spacer peptide consisting of 9 amino acids, and separating into a 25 kDa $\alpha$-subunit and a 58 kDa $\beta$-subunit. One $\alpha$-subunit is bound to one $\beta$-subunit through hydrophobic interactions, to form an about 83 kDa heterodimer having acylase activity. As is generally known, the first codon (ATG) encoding the N-terminal methionine is needed for translation initiation during protein synthesis in a prokaryote. The methionine is removed after the translation.

The polynucleotide encoding the mutant type II beta-lactam acylase or the alpha-subunit or the beta-subunit according to the present invention can be any polynucleotide that encodes the proper amino acid sequence according to the invention. Alternatively, the polynucleotide of the invention may comprise a coding sequence in which the codon usage for the various amino acids deviates from the codon usage in *Pseudomonas.* For example, the codon usage may be adapted to the codon usage of a particular host cell, which will or has been transformed with the DNA fragment encoding the altered type II beta-lactam acylase.

In a third aspect, the invention provides an expression vector or expression cassette comprising the polynucleotide of the invention as defined hereinbefore.

In a fourth aspect, the invention provides a transformed host cell, transformed with the polynucleotide of the invention or the expression vector or expression cassette of the invention. The transformed host cell may be used for the production of the mutant type II beta-lactam acylase of the invention.

Host cells for the production of the mutant type II beta-lactam acylase of the invention are preferably host cells which are known in the art for their efficient protein or enzyme production, either extracellular or intracellularly, for example microorganisms such as fungi, yeast and bacteria. Examples of preferred host cells comprise, but are not limited to, the following genera: *Aspergillus* (e.g. *A. niger, A. oryzea*), *Penicillium* (e.g. *P. emersonii, P. chrysogenum*), *Saccharomyces* (e.g. *S. cerevisiae*), *Kluyveromyces* (e.g. *K. lactis*), *Bacillus* (e.g. *B. subtilis, B. licheniformis, B. amyloliquefaciens*). *Escherichia,* (*E. coli*), *Streptomyces* (e.g. *S. clavuligerus*), Pseudomonas.

In a fifth aspect, the invention provides a process for the production of the mutant type II beta-lactam acylase of the invention comprising cultivating the transformed host cell according to the invention under conditions conducive to the production of the mutant expandase and, optionally, recovering the mutant expandase.

In a sixth aspect, the invention provides a process for the production of a deacylated beta-lactam compound of interest comprising the step of deacylating an acylated precursor of the beta-lactam compound of interest using the mutant type II beta-lactam acylase of the invention. Deacylated beta-lactam compounds of interest may be derivates of naturally occurring penicillins or cephalosporins such as 6-APA, 7-ACA, 7-ADCA, 7-ADAC, 7-amino-3-carbamoyloxymethyl-3-cephem-4-carboxylic acid (e.g. WO2004/106347) and others. Preferably, the deacylated beta-lactam compound of interest is 7-ADCA or 7-ACA, most preferred is 7-ADCA. Acylated precursors of the beta-lactam compound of interest may have an acyl belonging to the group of consisting of dicarboxylic acids. Preferred acyl groups are succinyl, glutaryl, adipyl, alpha-ketoadipyl and aminoadipyl. More preferred are adipyl and aminoadipyl, highly preferred is adipyl. Preferred acylated precursors of beta-lactam compound of interest are adipyl-7-ADCA, adipyl-7-ACA, aminoadipyl-7-ADCA and aminoadipyl-7-ACA, the latter known as CEFC; most preferred is adipyl-7-ADCA.

The process of the invention for the production of a deacylated beta-lactam compound of interest may be carried out in a batchwise mode whereby the mutant type II beta-lactam acylase is used in a dissolved state in a solution comprising the acylated precursor of the beta-lactam compound of interest.

More preferably, the mutant type II beta-lactam acylase is used as an immobilized form. The advantage thereof is that the mutant type II beta-lactam acylase may be recovered after completion of the deacylation reaction and be reused for further deacylation reactions. In this way, the cost in use of the mutant type II beta-lactam acylase can be reduced significantly, thus increasing the economic attractiveness of the deacylation process. Conditions for the deacylation reaction as well as the immobilization of the enzyme are known in the prior art (e.g. Kallenberg, A.I. et al. Adv. Synth. Catal. (2005), 347, 905-926).

In a seventh aspect, the present invention relates to the use of the mutant type II beta-lactam acylase of the invention in a process for the production of a deacylated beta-lactam compound of interest which process comprises the step of deacylating an acylated precursor of the beta-lactam compound of interest. Deacylated beta-lactam compounds of interest may be derivates of naturally occurring penicillins or cephalosporins such as 6-APA, 7-ACA, 7-ADCA, 7-ADAC, 7-amino-3-carbamoyloxymethyl-3-cephem-4-carboxylic acid and others. Preferably, the deacylated beta-lactam compound of interest is 7-ADCA or 7-ACA, most preferred is 7-ADCA. Acylated precursors of the beta-lactam compound of interest may have an acyl belonging to the group of consisting of dicarboxylic acids. Preferred acyl groups are succinyl, glutaryl, adipyl, alpha-ketoadipyland aminoadipyl. More preferred are adipyl and aminoadipyl, highly preferred is adipyl. The most preferred acylated precursors of the beta-lactam compound of interest are adipyl-7-ADCA, adipyl-7-ACA, adipyl-7-amino-3-carbamoyloxymethyl-3-cephem-4-carboxylic acid, alpha-ketoadipyl-7-ADCA, alpha-ketoadipyl-7-ACA, aminoadipyl-7-ADCA, aminoadipyl-7-ACA, the latter known as CEFC. The process of the invention for the production of a deacylated beta-lactam compound of interest may be carried out in a batchwise mode whereby the mutant type II beta-lactam acylase is used in a dissolved state in a solution comprising the acylated precursor of the beta-lactam compound of interest. More preferably, the mutant type II beta-lactam acylase is used in an immobilized form.

## MATERIALS AND METHODS

### *Preparation of acylase*

Plasmids with the wt gene or mutant genes were transformed to E.coli Top 10 cells (Invitrogen). The cells were inoculated in 100 ml flasks using 20 ml 2xTY medium containing 50 μg/ml zeocine at 37°C and 280 rpm. After 24 hours flasks with 100 ml 2xTY medium, 50 μg/ml zeocine and 0,05% arabinose were inoculated with 50 μl of the culture 1:1000 and grown at 25°C and 280 rpm. The cultures were centrifuged and frozen at -20°C. In order to prepare a cell free extract, the pellets were resuspended in extraction buffer (50mM Tris/HCL, 0.1 mg/ml DNAse1, 2mg/ml Lysozyme, 10mM DTT (dithiothreitol), 5mM MgSO4) and incubated. After 30 minutes the extract was centrifuged and the supernatant containing the acylase activity was used for activity measurements.

The acylase content was determined using SDS-PAGE gel electrophoresis and analytical HPLC size exclusion chromatography performed on a TSK 3000SWxl column with 0.1M phosphate buffer pH 7.0 as an eluent. Applied chromatographic conditions: flow rate 1.0 ml/min and detection at 280 nm. By comparing the observed areas of the acylase peaks one can compare the protein content of different samples. The acylase protein content is calculated from the OD280 using a molar extinction coefficient of 154350 ($M^{-1}.cm^{-1}$). In case of additional peaks in the HPLC chromatogram, the E280 value of the sample is corrected for the contribution of the additional peaks.

### *Purification*

Cell pellets from 100 ml cultures were resuspended with 1 ml of 20 mM Tris pH 8. After 9x10 seconds of sonification (Soniprep 150 I-BU03) with an amplitude of 10μ on ice (15 second breaks) the cell suspension was centrifuged for 5 minutes at 14000 rpm and 4°C in micro-centrifuge tubes. After the supernatant was brought to pH of 5.3-5.4 with 0.1 M HCl, it was centrifuged to remove the precipitate. Subsequently the supernatant was titrated back to pH=8 with NaOH. About 100-400μl was applied to a 1 ml MonoQ column, which was equilibrated with 20 mM Tris pH8 containing 10% NaCl. Buffer A (20 mM Tris pH8) and buffer B (20 mM Tris pH8 + 1 M NaCl) were mixed as followed during elution: minutes 0-1 10%B/90%A; 1-5 20%B/80%A; 5-9 40%B/60%A; 9-12 60%B/40%A; 12-15 100%B. The peak fractions containing acylase activity were collected and applied to a gel filtration column, TSKGel 3000SWxl, which was equilibrated with 100mM sodium phosphate buffer pH7. Peak fractions were collected and stored for further use.

***Reagents***

Adipyl-7ADCA can be prepared from adipic acid and 7-ADCA by enzymatic synthesis as described in WO9848037. In addition Adipyl-7ADCA can be prepared by chemical synthesis as described by Shibuya et al. in Agric. Biol. Chem., 1981, 45(7), 1561-1567 starting from adipic anhydride instead of glutaric anhydride.

An 8% (w/v) stock solution of adipyl-7ADCA substrate was prepared in the appropriate buffer and adjusted to the desired pH with 4 N NaOH.

The colour reagent 4-(dimethylamino)-benzaldehyde (p-DMBA) was freshly prepared by dissolving 200 mg in 100ml citric acid (315.5 g citric acid monohydrate dissolved in 1 liter ethanol).

Activity measurements in the range pH=8.0 to pH=10.0 were carried out in 0.2 M CHES buffer (2-(N-Cyclohexylamino)ethane sulfonic acid) adjusted to the desired pH with 4N HCl or 4N NaOH as needed.

***Measurement of acylase activity***

180 μl of the appropriate buffer was mixed with 200 μl of the substrate stock solution in the corresponding buffer and 20 μl enzyme solution and incubated during 20 minutes at the desired temperature, usually room temperature unless stated otherwise. Adding 600 μl colour reagent stopped the reaction. After 10 minutes at room temperature the absorbance was measured at 415 nm. The blanc measurements were done by adding colour solution to the assay before the enzyme is added. The acylase activity is calculated as the increase in optical density (OD) per minute (delta OD/min). In order to calculate absolute activities a 7-ADCA calibration line in the range 0.1 to 1 g 7-ADCA per liter was used.

***$K_M$ determinations and pH curves***

$K_M$ determinations were carried out using the assay as described. However the adipyl-7-ADCA concentration was varied from 0.5 to 4% (w/v) adipyl-7ADCA.

**EXAMPLES**

**Example 1**

**Acylase activity of SE83 ACYii mutants**

The acylase activity of the mutants with adipyl-7-ADCA as a substrate was determined at pH=8.5 and pH=9.5. Results are shown in Table 2.

Table 2: Relative activities of wild type and mutant acylases on adipyl-7ADCA setting the activity of wild type at pH=8.5 to 1. In parentheses the activities relative to wild type activity at pH=9.5. The assay was carried out at room temperature. Initial rates were measured in the presence of 4% (w/v) adipyl-7ADCA.

| Enzyme | Code | Acylase activity | | $\frac{\text{Activity at pH=9.5}}{\text{Activity at pH= 8.5}}$ |
|---|---|---|---|---|
| | | pH=8.5 | pH=9.5 | |
| Wild type | SE83 ACY-ii | 1.00 | 0.61 (1.00) | 0.61 (1.00) |
| Mutant | L161T | 1.95 | 1.38 (2.26) | 0.71 (1.16) |
| | H296Q | 1.35 | 1.11 (1.82) | 0.83 (1.36) |
| | L161S+E442G | 1.68 | 1.22 (2.00) | 0.73 (1.20) |
| | L161S+H589R | 1.60 | 1.05 (1.72) | 0.66 (1.08) |

At pH=8.5 as well as pH=9.5 the activity of the mutants is significantly higher compared to the wild type acylase. In addition, when comparing the activity at pH=9.5 with the activity at pH=8.5 it becomes clear that the activity of the mutant acylase at pH 9.5 is relatively higher compared to wild type. The pH activity profile of the mutants has been shifted to higher pH, making these mutants in particular suitable for use at elevated pH. This is of particular importance, because the yield of the conversion will increase at higher pH due to shifting the thermodynamic equilibrium further towards completion of the hydrolysis reaction.

Since during the process of conversion of adipyl-7ADCA into 7-ADCA and adipic acid, the concentration of the latter will increase, product inhibition could reduce the improvements of the mutants measured under conditions of initial rate. Therefore, the activity of wild type and mutant acylases was measured in the presence of 1.5% (w/v) adipic acid. Table 3 shows that under these conditions the activity of the mutants is significantly higher compared to the wild type acylase at pH=8.5 as well as pH=9.5. The pH activity profile for these mutants has not been shifted to higher pH.

The acylase activity of wild type and mutant acylases using adipyl-7-ADCA as a substrate was determined at pH=8.6, pH=9.1 and pH=9.5 in the presence of 1.5% (w/v) adipic acid. Results are shown in Table 4.

Table 4 shows that at pH=8.6, pH=9.1 as well as pH=9.5 the activity of the mutants is significantly higher compared to the wild type acylase. When comparing the activity improvement at pH=9.1 with the activity improvement at pH=8.6 (columns with ratio of activities) it becomes clear that the activity of the mutant acylases at pH 9.1 is relatively more improved compared to wild type. The pH activity profile of the mutants has been shifted to higher pH, making these mutants in particular suitable for use at elevated pH. When comparing pH=9.5 with pH=8.6 the activity of most mutant acylases is still more improved at pH=9.5 than at pH=8.5 compared to wild type.

Table 3: Relative activities of wild type and mutant acylases on adipyl-7ADCA setting the activity of wild type at pH=8.5 to 1. In parentheses the activities relative to wild type activity at pH=9.5. The assay was carried out at room temperature. The initial substrate concentration was 4% (w/v) adipyl-7ADCA. Initial activity was measured in the presence of 1.5% (w/v) adipic acid.

| Enzyme | Code | Acylase activity | | $\frac{\text{Activity at pH=9.5}}{\text{Activity at pH= 8.5}}$ |
|---|---|---|---|---|
| | | pH=8.5 | pH=9.5 | |
| Wild type | SE83 ACY-ii | 1.00 | 0.80 (1.00) | 0.80 (1.00) |
| Mutant | L161G | 2.53 | 1.80 (2.25) | 0.71 (0.89) |
| | L161G+ E10K | 2.13 | 1.47 (1.83) | 0.69 (0.86) |

Table 4: Relative activities of wild type and mutant acylases on adipyl-7ADCA setting the activity of wild type at pH=8.6 to 1. In parentheses the activities relative to wild type activity at pH=9.1 and pH=9.5 respectively. The assay was carried out at room temperature. The initial substrate concentration was 2% (w/v) adipyl-7ADCA. Initial activity was measured in the presence of 1.5% (w/v) adipic acid.

| Enzyme / Mutant | Relative Activity at pH | | | Ratio of activities at | |
|---|---|---|---|---|---|
| | 8.6 | 9.1 | 9.5 | $\frac{\text{pH=9.1}}{\text{pH=8.6}}$ | $\frac{\text{pH=9.5}}{\text{pH=8.6}}$ |
| Wild type SE83 ACY-ii | 1.00 | 0.76 (1.00) | 0.94 (1.00) | 0.76 (1.00) | 0.94 (1.00) |
| L161S | 2.23 | 2.24 (2.94) | 2.02 (2.13) | 1.01 (1.32) | 0.90 (0.96) |
| L161T | 2.08 | 2.57 (3.36) | 1.89 (2.00) | 1.23 (1.62) | 0.91 (0.96) |
| L161T+S29N | 2.28 | 2.82 (3.69) | 2.61 (2.76) | 1.23 (1.62) | 1.14 (1.21) |
| L161T+H274L | 3.58 | 5.59 (7.32) | 6.27 (6.63) | 1.56 (2.04) | 1.75 (1.85) |
| L161T+S29N+H274L | 4.46 | 6.63 (8.68) | 7.29 (7.72) | 1.49 (1.94) | 1.63 (1.73) |

(continued)

| Enzyme / Mutant | Relative Activity at pH | | | Ratio of activities at | |
|---|---|---|---|---|---|
| | 8.6 | 9.1 | 9.5 | $\frac{pH=9.1}{pH=8.6}$ | $\frac{pH=9.5}{pH=8.6}$ |
| L161T+1314V | 2.93 | 3.54 (4.63) | 3.39 (3.58) | 1.21 (1.58) | 1.16 (1.22) |
| L161T+N694T | 2.39 | 1.91 (2.49) | 1.48 (1.57) | 0.80 (1.04) | 0.62 (0.65) |
| L161T+V726I | 2.55 | 4.06 (5.32) | 4.25 (4.50) | 1.59 (2.09) | 1.67 (1.76) |
| L161T+S29N | 2.28 | 2.82 (3.69) | 2.61 (2.76) | 1.23 (1.62) | 1.14 (1.21) |
| L161T+Y706G | 4.51 | 4.05 (5.30) | 3.65 (3.86) | 0.90 (1.17) | 0.81 (0.86) |
| L161T+S29N+Y706G | 10.20 | 9.57 (12.53) | 8.33 (8.82) | 0.94 (1.23) | 0.82 (0.86) |
| L161T+S29N+P514Q | 3.08 | 3.16(4.13) | 2.98 (3.15) | 1.03 (1.34) | 0.97 (1.02) |
| L161T+S29N+H274L+ I314Q+N694T | 8.92 | 9.39 (12.29) | 9.69 (10.25) | 1.05 (1.38) | 1.09 (1.15) |
| L161T+S29N+R280Q+ I314V+A645T+V726I | 5.35 | 7.26 (9.51) | 7.20 (7.62) | 1.36 (1.78) | 1.34 (1.42) |
| L161T+S29F+H274L+ V726I | 4.30 | 7.23 (9.46) | 7.46 (7.89) | 1.68 (2.20) | 1.73 (1.84) |
| L161T+H274I+R280Q+ I314V | 6.06 | 9.05 (11.85) | 9.24 (9.78) | 1.49 (1.96) | 1.53 (1.61) |
| L161T+H274L+R589H | 3.00 | 3.72 (4.87) | 4.53 (4.79) | 1.24 (1.62) | 1.51 (1.60) |
| L161T+H274C+Y706G | 7.50 | 7.24 (9.47) | 7.60 (8.04) | 0.97 (1.26) | 1.01 (1.07) |
| L161S+H274T+R280Q + P514Q+V726I | 4.09 | 4.19 (5.48) | 4.82 (5.10) | 1.02 (1.34) | 1.18 (1.25) |

## Example 2

### Substrate affinity indicate by $K_M$ measurement of SE83 ACYii mutants

Table 5 shows the measured $K_M$ values for a number of mutants relative to wild type. The Michaelis constant $K_M$ represents the substrate concentration at which the enzyme operates at 50% of its maximal velocity. At substrate concentrations below the $K_M$ the enzyme becomes slower, at substrate concentrations above the $K_M$ the enzymes operates faster until at high substrate concentration the enzyme becomes fully saturated and operate at maximal velocity. At the end of an enzymatic conversion where the substrate gets exhausted a low $K_M$ is crucial in order to maintain substantial activity. In case the relative $K_M$ value for a mutant is <1.00, it means that at lower substrate concentrations, e.g. at the end of the conversion, the mutant has an advantage relative to wild type in maintaining a substantial higher activity.

Table 5: Relative substrate affinities represented as relative $K_M$ values. The $K_M$ determinations were done using the assay as described earlier. The adipyl-7ADCA concentration was varied from 0.5 to 4% Adipyl-7ADCA.

| | Relative $K_M$ at | |
|---|---|---|
| Enzyme / Mutant | pH = 8.6 | pH = 9.5 |
| Wt SE83 ACYii | 1.00 | 1.00 |
| L161T | 0.43 | 1.33 |
| L161S | 0.40 | 0.92 |
| L161T+N694T | 0.64 | 1.37 |
| L161T+I314V | 0.39 | 0.87 |
| L161T+1314V | 0.43 | 0.89 |
| S29N+L161T | 0.51 | 1.19 |
| S29N+L161T+P514Q | 0.57 | 1.31 |
| L161T+V726I | 0.24 | 0.62 |
| L161T+V726I | 0.21 | 0.64 |
| L161T+H274L | 0.18 | 0.48 |
| L161T+Y706G | 0.40 | 1.10 |

## Example 3

## Acylase activity of immobilized SE83 ACYii mutants

Immobilization was carried out as described in WO97/04086 using gelatin and chitosan as gelling agents and glutaric dialdehyde as cross-linking agent. The performance of the immobilized wild type acylase and mutant acylases was measured by performing a complete hydrolysis of adipyl-7ADCA in a temperature and pH controlled 100 ml reactor. Experiments were performed at 3.2% adipyl-7-ADCA. Immobilized enzyme was dosed in such a way that at least 90% conversion could be obtained within 120 minutes under the desired conditions. Conversions were carried out at pH=8.8 and 30°C and at pH=9.5 at 40°C. The same amount (in weight) of wild type and mutant acylase was used for the conversions. During the reaction the pH was kept constant by the addition of a solution of 1 M KOH. The activity of the immobilized acylase is expressed as ml KOH per min. In Figures 2a and 2b the rate, expressed as ml KOH per min, as a function of the conversion is shown.

The average of 6 runs was taken at pH=8.8 and 30°C. The data of the first 30% conversion was not included because the system was not completely stabilized, giving rise to a large scattering of the data. At pH=9.5 and 40°C the average of two runs was taken. Therefore, the variation is higher. However, the calculated slope gives a good indication of the activity. Figures 2a and 2b show that the activity of the mutant acylase is significantly higher during the whole conversion.

The stability of the immobilized acylases was determined by measuring 20 subsequent conversions of 180 minutes with the same batch of immobilized acylase. The rate between 30 and 50% conversion of each incubation was measured. The residual activity of an immobilized acylase is defined herein as the activity of the $20^{th}$ incubation compared to the rate of the first incubation.

Table 6 summarizes the results. It was observed that in particular under the conditions that shift the thermodynamic equilibrium of the hydrolysis reaction to a more complete conversion (high temperature and high pH) the stability of the mutated acylase is significantly improved compared to the wild type.

As a consequence of this higher hydrolytic activity and higher stability of the mutated acylase, the productivity per gram of the mutated acylase enzyme was increased considerably.

Table 6: Residual activity after 20 conversions at the conditions indicated.

| Reaction Condition | Acylase | Residual Activity (%) |
|---|---|---|
| pH 8.8; 30 °C | wild type | 103% |
| | L161T | 106% |
| pH 9.5; 30 °C | wild type | 98% |
| | L161T | 102% |
| pH 8.8; 40 °C | wild type | 88% |
| | L161T | 99% |
| pH 9.5; 40°C | wild type | 66% |
| | L161T | 76% |

## Claims

1. A mutant type II beta-lactam acylase selected from the group consisting of the SE83-acylI acylase of *Pseudomonas* as depicted in SEQ ID NO: 1, N176 from *Brevundimonas diminuta* as depicted in SEQ ID NO: 2 and the V22 acylase from *Brevundimonas diminuta* as depicted in SEQ ID NO: 3 and polypeptides with type II beta-lactam acylase activity having an amino acid sequence that is at least 90% identical over its entire length to SEQ ID NO: 1, SEQ ID NO: 2 or SEQ ID NO: 3 wherein said mutant type II beta-lactam acylase is **characterized in that** leucine at position 161 has been modified into glycine, serine or threonine.

2. Mutant type II beta-lactam acylase according to claim 1 comprising a modification at a position selected from the group consisting of 10, 29, 270, 274, 280, 296, 314, 442, 514, 589, 645, 694, 706 and 726 using the amino acid position numbering of the amino acid sequence of the SE83-acylI acylase of *Pseudomonas* as depicted in SEQ ID NO: 1.

3. Mutant type II beta-lactam acylase according to claim 2 wherein serine at position 29 has been modified into arginine, asparagine, glutamine or lysine and wherein tyrosine at position 706 has been modified into alanine, cysteine, glycine, proline, serine, threonine or valine.

4. Mutant type II beta-lactam acylase according to any one of claims 1-3 wherein said mutant beta-lactam acylase has an at least 1.5-fold improved in vitro beta-lactam acylase activity towards adipyl-7-ADCA in comparison with the model polypeptide with beta-lactam acylase activity.

5. A polynucleotide encoding the mutant type II beta-lactam acylase of anyone of claims 1-4.

6. An expression vector or cassette comprising the polynucleotide of claim 5.

7. A host cell comprising the polynucleotide of claim 5 or the vector or cassette of claim 6.

8. A method of producing the mutant type II beta-lactam acylase of any one of claims 1-4 comprising cultivating a host cell according to claim 7 under conditions conducive to the production of the mutant type II beta-lactam acylase and recovering the polypeptide.

9. A process for the production of 6-APA, 7-ACA, 7-ADCA, 7-ADAC or 7-amino-3-carbamoyloxymethyl-3-cephem-4-carboxylic acid comprising deacylating an acylated precursor of 6-APA, 7-ACA, 7-ADCA, 7-ADAC or 7-amino-3-carbamoyloxymethyl-3-cephem-4-carboxylic acid, respectively using the mutant type II beta-lactam acylase of any one of claims 1-4 wherein the acyl group of said acylated precursor is a dicarboxylic acid.

10. Process according to claim 9 wherein the mutant type II beta-lactam acylase is used in an immobilized form.

11. Use of the mutant type II beta-lactam acylase of claims 1-4 for the deacylation of an acylated precursor of 6-APA, 7-ACA, 7-ADCA, 7-ADAC or 7-amino-3-carbamoyloxymethyl-3-cephem-4-carboxylic acid.

12. Use according to claim 11 wherein the acyl group of said acylated precursor of 6-APA, 7-ACA, 7-ADCA, 7-ADAC or 7-amino-3-carbamoyloxymethyl-3-cephem-4-carboxylic acid is a dicarboxylic acid.

13. Use according to any of claims 11-12 wherein the mutant type II beta-lactam acylase is used in an immobilized form.

## Patentansprüche

1. Typ-II-beta-Lactamacylase-mutante, ausgewählt aus der Gruppe bestehend aus der SE83-acylI-Acylase aus *Pseudomonas* gemäß SEQ ID NO: 1, N176-Acylase aus *Brevundimonas diminuta* gemäß SEQ ID NO: 2 und der V22-Acylase aus *Brevundimonas diminuta* gemäß SEQ ID NO: 3 und Polypeptiden mit Typ-II-beta-Lactamacylaseaktivität mit einer Aminosäuresequenz mit mindestens 90% Identität über ihre gesamte Länge zu SEQ ID NO: 1, SEQ ID NO: 2 oder SEQ ID NO: 3, wobei die Typ-II-beta-Lactamacylase-mutante **dadurch gekennzeichnet ist, dass** das Leucin in Position 161 zu Glycin, Serin oder Threonin modifiziert worden ist.

2. Typ-II-beta-Lactamacylase-mutante nach Anspruch 1, umfassend eine Modifikation an einer Position, ausgewählt aus der Gruppe bestehend aus 10, 29, 270, 274, 280, 296, 314, 442, 514, 589, 645, 694, 706 und 726 unter Verwendung der Aminosäurepositionsnumerierung der Aminosäuresequenz der SE83-acyII-Acylase von *Pseudomonas* gemäß SEQ ID NO: 1.

**3.** Typ-II-beta-Lactamacylase-mutante nach Anspruch 2, wobei das Serin in Position 29 zu Arginin, Asparagin, Glutamin oder Lysin modifiziert worden ist und wobei das Tyrosin in Position 706 zu Alanin, Cystein, Glycin, Prolin, Serin, Threonin oder Valin modifiziert worden ist.

**4.** Typ-II-beta-Lactamacylase-mutante nach einem der Ansprüche 1-3, wobei die beta-Lactamacylase-Mutante im Vergleich zu dem Modellpolypeptid mit beta-Lactamacylaseaktivität eine mindestens um das 1,5-Fache verbesserte in-vitro-beta-Lactamacylase-aktivität gegenüber Adipyl-7-ADCA aufweist.

**5.** Polynukleotid, das für die Typ-II-beta-Lactamacylase-mutante nach einem der Ansprüche 1-4 codiert.

**6.** Expressionsvektor oder -kassette, umfassend das Polynukleotid nach Anspruch 5.

**7.** Wirtszelle, umfassend das Polynukleotid nach Anspruch 5 oder den Vektor oder die Kassette nach Anspruch 6.

**8.** Verfahren zur Herstellung der Typ-II-beta-Lactamacylase-mutante nach einem der Ansprüche 1-4, bei dem man eine Wirtszelle nach Anspruch 7 unter Bedingungen, die der Produktion der Typ-II-beta-Lactamacylase-mutante zuträglich sind, kultiviert und das Polypeptid gewinnt.

**9.** Verfahren zur Herstellung von 6-APA, 7-ACA, 7-ADCA, 7-ADAC oder 7-Amino-3-carbamoyloxymethyl-3-cephem-4-carbonsäure, bei dem man eine acylierte Vorstufe von 6-APA, 7-ACA, 7ADCA- 7-ADAC, 7-Amino-3-carbamoyloxymethyl-3-cephem-4-carbonsäure unter Verwendung der Typ-II-beta-Lactamacylase-mutante nach einem der Ansprüche 1-4 deacyliert, wobei es sich bei der Acylgruppe der acylierten Vorstufe um eine Dicarbonsäure handelt.

**10.** Verfahren nach Anspruch 9, wobei die Typ-II-beta-Lactamacylase-mutante in einer immobilisierten Form verwendet wird.

**11.** Verwendung der Typ-II-beta-Lactamacylase-mutante nach den Ansprüchen 1-4 zum Deacylieren einer acylierten Vorstufe von 6-APA, 7-ACA, 7-ADCA, 7-ADAC oder 7-Amino-3-carbamoyloxymethyl-3-cephem-4-carbonsäure.

**12.** Verwendung nach Anspruch 11, wobei es sich bei der Acylgruppe der acylierten Vorstufe von 6-APA, 7-ACA, 7-ADCA, 7-ADAC oder 7-Amino-3-carbamoyloxy-methyl-3-cephem-4-carbonsäure um eine Dicarbonsäure handelt.

**13.** Verwendung nach einem der Ansprüche 11-12, wobei die Typ-II-beta-Lactamacylase-mutante in einer immobilisierten Form verwendet wird.

## Revendications

**1.** Bêta-lactamine acylase de type II mutante, choisie dans le groupe constitué par la SE83-acyII acylase de *Pseudomonas* telle que décrite dans la SEQ ID n° : 1, la N176 acylase provenant de *Brevundimonas diminuta* telle que décrite dans la SEQ ID n° : 2 et la V22 acylase provenant de *Brevundimonas diminuta* telle que décrite dans la SEQ ID n° : 3 ainsi que des polypeptides avec une activité de bêta-lactamine acylase de type II ayant une séquence d'acides aminés qui est identique, au moins à 90% et sur sa longueur totale, à la SEQ ID n° : 1, SEQ ID n° : 2 ou SEQ ID n° : 3, dans laquelle ladite bêta-lactamine acylase de type II mutante est **caractérisée en ce qu'**une leucine en position 161 a été modifiée en une glycine, sérine ou thréonine.

**2.** Bêta-lactamine acylase de type II mutante, selon la revendication 1, comprenant une modification au niveau d'une position choisie dans le groupe constitué par les 10, 29, 270, 274, 280, 296, 314, 442, 514, 589, 645, 694, 706 et 726, en utilisant la numérotation de la position d'acide aminé de la séquence d'acides aminés de la SE83-acyII acylase de *Pseudomonas,* telle que décrite dans la SEQ ID n° : 1.

**3.** Bêta-lactamine acylase de type II mutante selon la revendication 2, dans laquelle une sérine en position 29 a été modifiée en une arginine, asparagine, glutamine ou lysine et dans laquelle une tyrosine en position 706 a été modifiée en une alanine, cystéine, glycine, proline, sérine, thréonine ou valine.

4. Bêta-lactamine acylase de type II mutante selon l'une quelconque des revendications 1 à 3, dans laquelle ladite bêta-lactamine acylase mutante a une activité de bêta-lactamine acylase améliorée, d'au moins 1,5 fois *in vitro*, envers l'adipyl-7-ADCA en comparaison avec le polypeptide modèle avec une activité de bêta-lactamine acylase.

5. Polynucléotide codant pour la bêta-lactamine acylase de type II mutante selon l'une quelconque des revendications 1 à 4.

6. Vecteur ou cassette d'expression comprenant le polynucléotide selon la revendication 5.

7. Cellule hôte comprenant le polynucléotide, selon la revendication 5, ou bien le vecteur ou la cassette selon la revendication 6.

8. Procédé de production de la bêta-lactamine acylase de type II mutante, selon l'une quelconque des revendications 1 à 4, comprenant les étapes consistant à cultiver une cellule hôte, selon la revendication 7, dans des conditions qui conduisent à la production de la bêta-lactamine acylase de type II mutante, et récupérer le polypeptide.

9. Processus pour la production de 6-APA, 7-ACA, 7-ADCA, 7-ADAC ou d'acide 7-amino-3-carbamoyloxyméthyl-3-céphem-4-carboxylique comprenant la désacylation d'un précurseur acylé des 6-APA, 7-ACA, 7-ADCA, 7-ADAC ou acide 7-amino-3-carbamoyloxyméthyl-3-céphem-4-carboxylique respectivement, en utilisant la bêta-lactamine acylase de type II mutante, selon l'une quelconque des revendications 1 à 4, dans laquelle le groupe acyle dudit précurseur acylé est un acide dicarboxylique.

10. Processus selon la revendication 9, dans laquelle la bêta-lactamine acylase de type II mutante est utilisée sous une forme immobilisée.

11. Utilisation de la bêta-lactamine acylase de type II mutante, selon l'une quelconque des revendications 1 à 4, pour la désacylation d'un précurseur acylé des 6-APA, 7-ACA, 7-ADCA, 7-ADAC ou acide 7-amino-3-carbamoyloxyméthyl-3-céphem-4-carboxylique.

12. Utilisation selon la revendication 11, dans laquelle le groupe acyle dudit précurseur acylé des 6-APA, 7-ACA, 7-ADCA, 7-ADAC ou acide 7-amino-3-carbamoyloxyméthyl-3-céphem-4-carboxylique est un acide dicarboxylique.

13. Utilisation selon l'une quelconque des revendications 11 à 12, dans laquelle la bêta-lactamine acylase de type II mutante est utilisée sous une forme immobilisée.

```
SE83_ACYII    MTMAAKTDREALQAALPPLSGSLSIPGLSAPVRVQRDGWGIPHIKASGEADAYRALGFVH 60
N176_CCAC     MTMAANTDRAVLQAALPPLSGSLPIPGLSASVRVRRDAWGIPHIKASGEADAYRALGFVH 60
V22_CCAC      MTMAANTDRAVLQAALPPLSGSLPIPGLSASVPIQRDAWGIPHIKASGEADAYRALGFVH 60
              *****:*** .************.******.* ::**.**********************

SE83_ACYII    AQDRLFQMELTRRKALGRAAEWLGAEAAEADILVRRLGMEKVCRRDFEALGAEAKDMLRA 120
N176_CCAC     SQDRLFQMELTRRKALGRAAEWLGAEAAEADILVRRLGMEKVCRRDFEALGVEAKDMLRA 120
V22_CCAC      AQDRLFQMELTRRKALGRAAEWLGAEAAEADILVRRLGMEKVCRRDFEALGAEAKDMLRA 120
              :**************************************************.********

SE83_ACYII    YVAGVNAFLASGAPLPIEYGLLGAEPEPWEPWHSIAVMRRLGLLMGSVWFKLWRMLALPV 180
N176_CCAC     YVAGVNAFLASGAPLPVEYGLLGAEPEPWEPWHSIAVMRRLGLLMGSVWFKLWRILALPV 180
V22_CCAC      YVAGVNAFLASGVPLPVEYGLLGAEPEPWEPWHSIAVMRRLGLLMGSVWFKLWRMLALPV 180
              ************.***:*************************************:*****

SE83_ACYII    VGAANALKLRYDDGGQDLLCIPPGVEAERLEADLAALRPAVDALLKAMGGDASDAAGGGS 240
N176_CCAC     VGAANALKLRYDDGGRDLLCIPPGAEADRLEADLATLRPAVDALLKAMGGDASDAAGGGS 240
V22_CCAC      VGAANALKLRYDDGGRDLLCIPPRAEADRLEADLATLRPAVDALLKAMGGDASDAAGGGS 240
              ***************:******* .**:*******:************************

SE83_ACYII    NNWAVAPGRTATGRPILAGDPHRVFEIPGMYAQHHLACDRFDMIGLTVPGVPGFPHFAHN 300
N176_CCAC     NNWAVAPGRTATGRPILAGDPHRVFEIPGMYAQHHLACDRFDMIGLTVPGVPGFPHFAHN 300
V22_CCAC      NNWAVAPGRTATGRPILAGDPHRVFQIPGMYAQHHLACDRFDMIGLTVPGVPGFPHFAHN 300
              *************************:**********************************

SE83_ACYII    GKVAYCVTHAFMDIHDLYLEQFAEDGRTARFGNEFEPVAWRRDRIAVRGGADREFDIVET 360
N176_CCAC     GKVAYCVTHAFMDIHDLYLEQFAGEGRTARFGNDFEPVAWSRDRIAVRGGADREFDIVET 360
V22_CCAC      GKVAYCVTHAFMDIHDLYLEQFAEEGRRARFGNDFEPAAWSRDRIAVRGGADREFDIIET 360
              *********************** :** *****:***.** ****************:**

SE83_ACYII    RHGPVIAGDPLEGAALTLRSVQFAETDLSFDCLTRMPGASTVAQLYDATRGWGLIDHNLV 420
N176_CCAC     RHGPVIAGDPRDGAALTLRSVQFAETDLSFDCLTRMPGASTVAQLYDATRGWGLIDHNLV 420
V22_CCAC      RHGPVIAGDPRDGAALTLRSVQFAETDLSFDCLTRMPGASTVAQLYDATRGWGLIDHNLV 420
              ********** :************************************************

SE83_ACYII    AGDVAGSIGHLVRARVPSRPRENGWLPVPGWSGEHEWRGWIPHEAMPRVIDPPGGLIVTA 480
N176_CCAC     AGDVAGSIGHLVRARVPSRPRENGWLPVPGWSGEHEWRGWIPHEAMPRVIDPPGGIIVTA 480
V22_CCAC      AGDVGGSIGHLVRARVPSRSRENGWLPVPGWSGEHEWRGWIPHEAMPRVIDPPGGIIVTA 480
              ****.**************.***********************************:****

SE83_ACYII    NNRVVADDHPDYLCTDCHPPYRAERIMERLVASPAFAVDDAAAIHADTLSPHVGLLRARL 540
N176_CCAC     NNRVVADDHPDYLCTDCHPPYRAERIMKRLVANPAFAVDDAAAIHADTLSPHVGLLRRRL 540
V22_CCAC      NNRVVADDHPDYLCTDCHPPYRAEPIMKRLVANPAFAVDDAAAIHADTLSPHVGLLRRRL 540
              ************************ **:****.************************ **

SE83_ACYII    EALGIQGSLPAEELRQTLIAWDGRMDAGSQAASAYNAFRRALTRLVTARSGLEQAIAHPF 600
N176_CCAC     EALGARDDSAAEGLRQMLVAWDGRMDAASEVASAYNAFRRALTRLVTDRSGLEQAISHPF 600
V22_CCAC      EALGARDDSAAEGLRQMLVAWDGRMDAASEVASAYNAFRRALTRLVTDRSGLEQAISHPF 600
              **** :.. .** *** *:********.*:.**************** ********:***

SE83_ACYII    AAVPPGVSPQGQVWWAVPTLLRNDDAGMLKGWSWDEALSEALSVATQNLTGRGWGEEHRP 660
N176_CCAC     AAVAPGVSPQGQVWWAVPTLLRDDDAGMLKGWSWDQALSEALSVASQNLTGRSWGEEHRP 660
V22_CCAC      AAVAPGVSPQGQVWWAVPTLLRDDDAGMLKGWSWDQALSEALSVASQNLSRRSWGEEHRP 660
```

```
                 ***.******************:************:*********:***: *.*******

SE83_ACYII       RFTHPLSAQFPAWAALLNPVSRPIGGDGDTVLANGLVPSAGPEATYGALSRYVFDVGNWD 720
N176_CCAC        RFTHPLATQFPAWAGLLNPASRPIGGDGDTVLANGLVPSAGPQATYGALSRYVFDVGNWD 720
V22_CCAC         RFTHPLATQFPAWAGLLNPASRPIGGDGDTVLANGLVPSAGPQATYGALSRYVFDVGNWD 720
                 ******::******.****.********************:*******************

SE83_ACYII       NSRWVVFHGASGHPASPHYADQNAPWSDCAMVPMLYSWDRIAAEAVTSQELVPA- 774
N176_CCAC        NSRWVVFHGASGHPASAHYADQNAPWSDCAMVPMLYSWDRIAAEAVTSQELVPA- 774
V22_CCAC         NSRWVVFHGASGHPASAHYADQNAPWSDCAMVPMLYSWDRIAAEAVTSQELVPAX 775
                 ****************.*************************************
```

Fig. 1

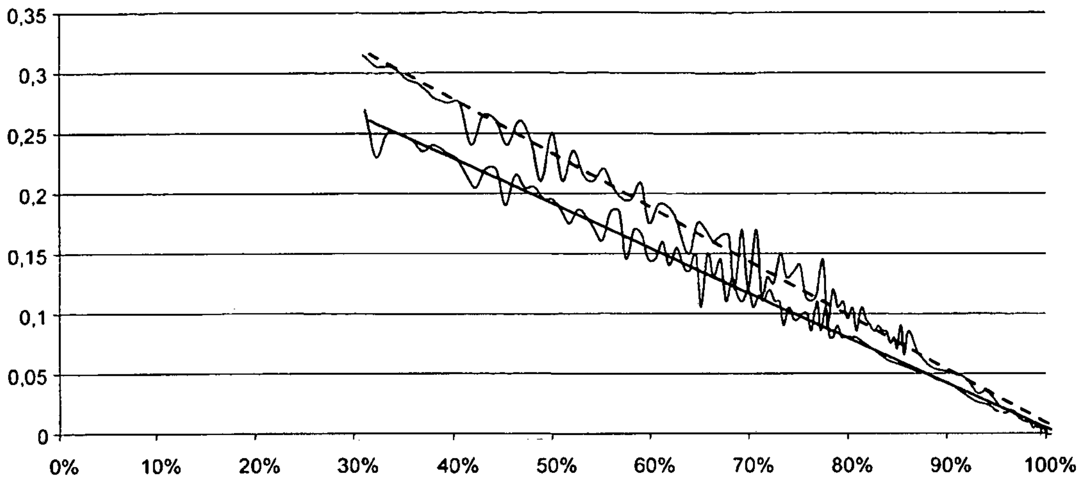
0,35
0,3
0,25
0,2
0,15
0,1
0,05
0
0%
10%
20%
30%
40%
50%
60%
70%
80%
90%
100%

Fig. 2a

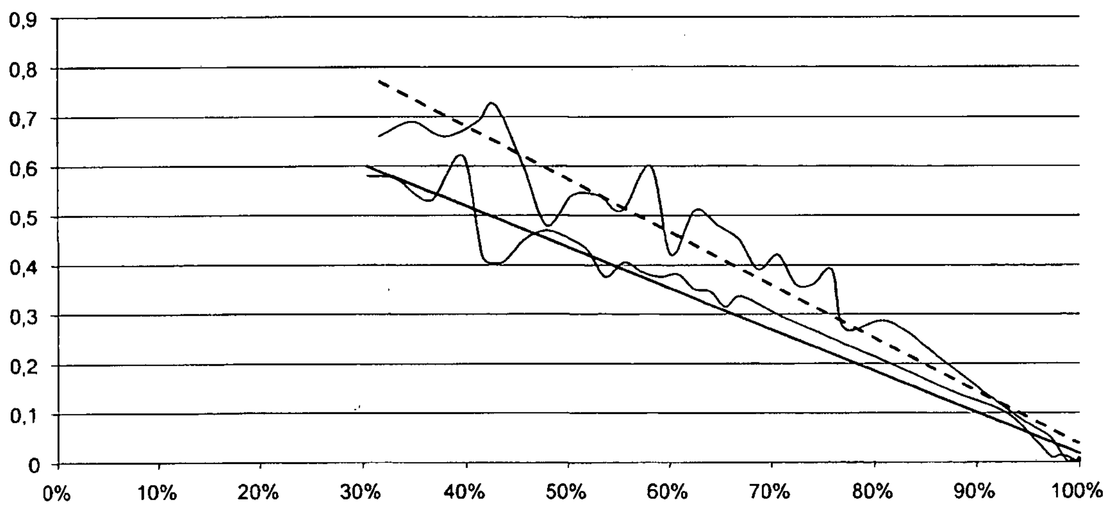
0,9
0,8
0,7
0,6
0,5
0,4
0,3
0,2
0,1
0
0%
10%
20%
30%
40%
50%
60%
70%
80%
90%
100%

Fig. 2b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0448180 A
- US 4003894 A
- WO 9305158 A
- WO 9504148 A
- WO 9504149 A
- WO 9116435 A
- WO 9512680 A
- WO 2005014821 A
- EP 1553175 A
- WO 2004106347 A
- WO 9848037 A
- WO 9704086 A


### Non-patent literature cited in the description


- **INGOLIA ; QUEENER.** *Med Res Rev,* 1989, vol. 9, 245-264
- **AHARONOWITZ ; COHEN ; MARTIN.** *Ann Rev Microbiol,* 1992, vol. 46, 461-495
- **ALVI et al.** *J Antibiot,* 1995, vol. 48, 338-340
- **CANTWELL et al.** *Proc R Soc Lond B,* 1992, vol. 248, 283-289
- Penicillin acylases and beta-lactamases'' In: ''Microbial Enzymes and Bioconversions. **VANDAMME E. J.** Economic Microbiology. Acad. Press, 1980, vol. 5, 467-552
- **OLLSON A.** *Appl. Environm. Microb.,* 1976, 203
- **MATSUDA et al.** *J. Bacteriol,* 1985, vol. 163, 1222
- *J. Biol.Chem.,* 2002, vol. 277, 2823
- **MATSUDA et al.** *J. Bacteriol,* 1987, vol. 169, 5815
- *J. Bacteriol.,* 1987, vol. 169, 5821
- **ARAMORI et al.** *Journal of Fermentation and Bioengineering,* 1991, vol. 72, 232-243
- **KALLENBERG, A.I. et al.** *Adv. Synth. Catal.,* 2005, vol. 347, 905-926
- **SHIBUYA et al.** starting from adipic anhydride instead of glutaric anhydride. *Agric. Biol. Chem.,* 1981, vol. 45 (7), 1561-1567